# EUROPÄISCHE PATENTSCHRIFT

(11) **EP 1 970 091 B1**
(45) Veröffentlichungstag und Bekanntmachung des Hinweises auf die Patenterteilung: **27.10.2010**
(21) Anmeldenummer: 07005217.0
(22) Anmeldetag: 14.03.2007
(51) Int. Cl.: A61M 25/06

(54) **Insertionskopf für medizinische oder pharmazeutische Anwendungen**
Insertion head for medical or pharmaceutical applications
Tête d'insertion pour applications médicales ou pharmaceutiques

(43) Veröffentlichungstag der Anmeldung: 17.09.2008
(73) Patentinhaber: F. Hoffmann-La Roche AG, 4070 Basel (CH); Roche Diagnostics GmbH, 68305 Mannheim (DE)
(72) Erfinder: Liniger, Jürg, 3072 Ostermundigen (CH); Wyss, Martin, 3510 Konolfingen (CH); Thalmann, Christian, 6356 Kersiten (CH); Weibel, Michael, 3422 Alchenflüh (CH)
(74) Vertreter: Schalch, Rainer

(56) Entgegenhaltungen:
- EP-A- 1 764 125
- WO-A-2006/015507
- FR-A1- 2 725 902
- US-A1- 2003 199 823

## Beschreibung

Die Erfindung betrifft einen Insertionskopf für medizinische oder pharmazeutische Anwendungen, der auf einem organischen Gewebe, vorzugsweise der menschlichen Haut, platzierbar ist und eine Einführeinrichtung aufweist, die in das Gewebe eindringt, wenn der Insertionskopf auf dem Gewebe platziert wird oder gegebenenfalls auch erst nachdem der Insertionskopf auf dem Gewebe platziert wurde. Der Insertionskopf kann insbesondere Bestandteil eines Infusionssets für die Verabreichung eines Medikaments sein.

Aus der DE 198 21 723 C1 ist ein Insertionskopf bekannt, der eine Basis, eine flexible Kanüle und eine Einstechnadel umfasst. Die Kanüle ragt von einer Unterseite der Basis ab. Die Einstechnadel stabilisiert die Kanüle, während die Kanüle in das Gewebe eines Patienten eingeführt wird. Für die Stabilisierung durchragt die Einstechnadel die Kanüle, und die Kanüle umschmiegt die Einstechnadel. Als Schutz vor Stichverletzungen ist an der Basis lösbar ein Nadelschutz befestigt. Die von der Unterseite des Insertionskopfs abragende Einstechnadel mit der umgebenden Kanüle und insbesondere auch der Nadelschutz vergrößern das Volumen des Insertionskopfs und dadurch auch dessen Verpackung beträchtlich. Ferner ist das Entfernen des Nadelschutzes umständlich und mit einer Verletzungsgefahr verbunden.

Die deutsche Patentanmeldung Nr. 10 2004 039 408.3 hat einen Insertionskopf zum Gegenstand, der eine Basis mit einer auf organischem Gewebe platzierbaren Unterseite und platzsparend eine in das Gewebe einführbare, von der Basis beweglich gelagerte Einführeinrichtung umfasst. Für die Lagerung, den Transport und die Handhabung bis zur Einführung in das Gewebe nimmt die Einführeinrichtung eine Schutzposition ein. Für das Einführen ist sie aus der Schutzposition in eine Einführposition bewegbar. Als bevorzugte Art der Bewegung wird eine Schwenkbeweglichkeit offenbart. Um die Bewegung der Einführeinrichtung bewirken zu können, ist für den Benutzer ein Griff vorgesehen, der gemeinsam mit der Einführeinrichtung schwenkbar ist. Der Insertionskopf ist vorteilhaft kompakt und erfordert auch nicht das Entfernen eines Nadelschutzes. Um die Einführeinrichtung in die Einführposition zu bewegen, muss der Benutzer jedoch mit der einen Hand den Insertionskopf greifen und mit der anderen Hand den Griff mit der Einführeinrichtung schwenken.

Es ist eine Aufgabe der Erfindung, einen Insertionskopf zu schaffen, der einen integrierten Schutz für eine Einführeinrichtung aufweist, beispielsweise dem Insertionskopf der deutschen Patentanmeldung Nr. 10 2004 039 408.3 vergleichbar, aber einfacher und insbesondere mit einer einzigen Hand handhabbar ist.

Aus EP 1 764 125 A1, ein Dokument das unter die Bestimmungen von Art. 54 (3) EPÜ fällt, ist ein Insertionskopf für medizinische oder pharmazeutische Anwendungen bekannt, welcher umfasst:
ein Gehäuse mit einem ersten Gehäuseteil und einem zweiten Gehäuseteil, wobei der erste und der zweite Gehäuseteil relativ zueinander bewegbar sind;
eine Basis;
eine flexible Einführeinrichtung, welche von einer Einstecheinrichtung stabilisiert ist, wobei die Einführeinrichtung und die Einstecheinrichtung gemeinsam bewegbar gelagert sind,
wobei die Einführeinrichtung und die Einstecheinrichtung relativ zum Gehäuse aus einer Schutzposition, in der die freien Enden der Einführeinrichtung und der Einstecheinrichtung innerhalb der Begrenzungen des Gehäuses und/oder der Basis angeordnet sind, in eine Einführposition bewegbar sind, in der die freien Enden aus den Begrenzungen des Gehäuses und/- oder der Basis hervorragen, derart, dass diese in ein organisches Gewebe eingeführt werden können, und eine Kopplung, welche die Relativbewegung der beiden Gehäuseteile zueinander in eine Bewegung der Einführeinrichtung und der Einstecheinrichtung überträgt, derart, dass diese durch Bewegen der beiden Gehäuseteile relativ zueinander von der Schutzposition in die Einführposition bewegbar sind,
wobei die Basis in der Einführposition eine auf dem organischen Gewebe platzierbare Unterseite aufweist, von welcher die Einführeinrichtung und die Einstecheinrichtung hervorstehen, zur Ermöglichung eines Einführens derselben in das Gewebe unter einem Platzieren der Basis mit der Unterseite auf dem Gewebe, wobei in der Einführposition die Einstecheinrichtung derartig mit dem Gehäuse verbunden ist und von der Einführeinrichtung lösbar ist, dass die Einstecheinrichtung nach dem Einführen der Einführeinrichtung und der Einstecheinrichtung in das Gewebe durch Ergreifen des Gehäuses mit einer Hand und Bewegen desselben mit der Hand in einer Richtung entgegengesetzt der bestimmungsgemässen Einsetzrichtung von der Basis und der Einführeinrichtung entfernt werden kann.

Es ist Aufgabe der Erfindung, einen Insertionskopf zur Verfügung zu stellen, bei dem die Einstecheinrichtung nach dem Entfernen von der Basis und der Einführeinrichtung nicht schutzlos freiliegt.

Der erfindungsgemässe Insertionskopf umfasst ein Gehäuse mit einem ersten Gehäuseteil und einem zweiten Gehäuseteil, welche relativ zueinander bewegbar sind. Bevorzugterweise ist das Gehäuse als Griff ausgebildet, wobei das erste Gehäuseteil eine erste Griffkomponente und das zweite Gehäuseteil eine zweite Griffkomponente bildet.

Weiter umfasst der Insertionskopf eine Basis sowie eine flexible Einführeinrichtung, welche von einer Einstecheinrichtung stabilisiert wird, um zu verhindern, dass die Einführeinrichtung beim Einführen in das Gewebe knickt. Die Einführeinrichtung kann beispielsweise als flexible Kanüle (Softkanüle) ausgebildet sein, und von einer biegesteifen Einstecheinrichtung, wie z.B. einer biegesteifen Kanüle oder Nadel, während der Einführung in das Gewebe stabilisiert werden, z.B. indem sie von dieser durchdrungen wird. Die Einführeinrichtung ist vorzugsweise in Einführrichtung lang gestreckt und vorzugsweise schlank.

Die Einführeinrichtung und die diese stabilisierende Einstecheinrichtung sind, bevorzugterweise innerhalb der Begrenzungen des Gehäuses, gemeinsam bewegbar gelagert, z.B. indem die Einführeinrichtung und/oder die Einsticheinrichtung in einem der beiden Gehäuseteile bewegbar gelagert sind. Dabei ist die Lagerung derartig ausgebildet, dass die Einführeinrichtung und die Einstecheinrichtung relativ zum Gehäuse aus einer Schutzposition, in weicher deren freie Enden, welche zum Einstechen in ein organisches Gewebe bevorzugterweise eines Menschen vorgesehen sind, zur Vermeidung eines unbeabsichtigten Inkontaktkommens mit dem Bediener oder der Umgebung innerhalb der Begrenzungen des Gehäuses und/oder der Basis angeordnet sind, in eine Einführposition bewegbar sind, in welcher diese freien Enden aus den Begrenzungen des Gehäuses und/oder der Basis hervorstehen, derart, dass diese in das organische Gewebe eingestochen werden können.

In der Schutzposition wird also ein unbeabsichtigtes Inkontaktkommen des Bedieners mit der Einführeinrichtung und der Einstecheinrichtung erschwert oder verunmöglicht, so dass eine Verletzung des Bedieners und eine Kontamination der Einführ- und Einstecheinrichtung beim Hantieren mit dem Insertionskopf in diesem Zustand sicher verhindert wird. Vorzugsweise befinden sich die Einführeinrichtung und die Einstecheinrichtung in der Schutzposition über ihre gesamte Länge innerhalb der Begrenzungen des Gehäuses und werden bevorzugterweise vollständig abgeschirmt, vorzugsweise auch blickdicht verdeckt. In bevorzugten Ausführungen weisen die Einführeinrichtung und die Einstecheinrichtung in der Schutzposition zumindest im Wesentlichen parallel zu einer Aussenfläche des Gehäuses. Dies begünstigt eine flache Bauweise des Insertionskopfes, wobei dessen Höhe rechtwinklig zu dieser Aussenfläche gemessen wird.

In der Einführposition, in welcher der Insertionskopf applikationsbereit ist, stehen also die freien Enden der Einführeinrichtung und der Einstecheinrichtung über die Begrenzungen des Gehäuses hervor, bevorzugterweise über dessen Unterseite hervor. Vorzugsweise ragen die Einführeinrichtung und die Einstecheinrichtung in der Einführungsposition von der Unterseite des Gehäuses vor. Grundsätzlich können diese jedoch auch von einer anderen Seite des Gehäuses vorragen, solange sie für ein Eindringen in das Gewebe über die Unterseite ausreichend weit vorragen. Die Einführeinrichtung ragt bevorzugterweise mit einer an subkutane Applikationen angepassten Länge über die Unterseite des Gehäuses vor, vorzugsweise unmittelbar von der Unterseite ab oder aus der Unterseite hervor. Für Applikationen innerhalb der Haut oder in intramuskulärem Gewebe ist die Einführeinrichtung entsprechend kürzer oder länger. Als Einführeinrichtung wird derjenige Längenabschnitt verstanden, der bei bestimmungsgemäss appliziertem Insertionskopf in das Gewebe ragt.

Weiter umfasst der erfindungsgemässe Insertionskopf eine mechanische Kopplung, welche eine Bewegung der beiden Gehäuseteile bzw. Griffkomponenten relativ zueinander in eine Bewegung der Einführ- und Einstecheinrichtung umwandelt, derartig, dass die Einführeinrichtung und die Einstecheinrichtung durch die Relativbewegung der beiden Gehäuseteile zueinander gemeinsam von der Schutzposition in die Einführposition bewegt werden können.

Die Basis stellt zumindest in der Einführposition eine auf dem organischen Gewebe platzierbare Unterseite bereit, bevorzugterweise die Unterseite des Gehäuses, welche gegebenenfalls auch von einem Pflaster, einem Klebepad oder einer Klebeschicht zur Befestigung auf der Oberfläche des Gewebes gebildet sein kann, und über welche in der Einführposition die Einführeinrichtung und die Einstecheinrichtung hervorstehen, so dass ein Einstechen bzw. Einführen derselben in das Gewebe erfolgen kann, unter einem Platzieren der Basis mit der Unterseite auf dem Gewebe.

Weiter ist der Insertionskopf derartig ausgebildet, dass die Einstecheinrichtung in der Einführposition derartig mit dem Gehäuse verbunden oder verbindbar und von der Einführeinrichtung lösbar ist, dass die Einstecheinrichtung nach dem Einführen der Einführeinrichtung und der Einstecheinrichtung in das Gewebe durch Ergreifen des Gehäuses mit einer Hand und Bewegen desselben mit der Hand in einer Richtung entgegengesetzt der Einführ- bzw. Einstechrichtung von der Basis und der Einführeinrichtung entfernt werden kann. Hierfür ist es beispielsweise vorgesehen, dass die Einstecheinrichtung Rastmittel aufweist, welche am Ende der Bewegung von der Schutzposition in die Einführposition, also beim Erreichen der Einführposition, mit zugeordneten Rastmitteln des Gehäuses verrasten und dadurch einen Formschluss entgegen der Einsetzrichtung erzeugen, während zumindest in der Einsetzposition die Verbindung zwischen Einführeinrichtung und Einstecheinrichtung in Richtung entgegen der Einsetzrichtung lediglich kraftschlüssig ist, z.B. infolge der Reibung der als Einstechnadel ausgebildeten Einstecheinrichtung in einem Septum eines der Einführeinrichtung zugeordneten Kanülengehäuses.

Weiter umfasst der erfindungsgemässe Insertionskopf Rückstellmittel, mittels welcher die Einstecheinrichtung direkt nach dem Entfernen von der Basis und der Einführeinrichtung, was durch Ergreifen des Gehäuses mit der Hand und Bewegen desselben in einer Richtung entgegengesetzt der Einführrichtung erfolgt, automatisch zurück in eine Schutzposition bringbar ist, in welcher das freie Ende der Einstecheinrichtung innerhalb der Begrenzungen des Gehäuses angeordnet ist, so dass ein unbeabsichtigtes Inkontaktkommen des Bedieners mit der nun kontaminierten Einstecheinrichtung wieder sicher verhindert wird. Dabei ist es bevorzugt, wenn der Insertionkopf derartig ausgebildet ist, dass die Einstecheinrichtung direkt nach dem Lösen von der Basis und der Einführeinrichtung zwangsläufig und ohne Einflussmöglichkeit des Bedieners in die Schutzposition gebracht wird, z.B. indem sie von einer Feder betätigt automatisch zurückgezogen oder zurückgeschwenkt wird. Alternativ ist es auch bevorzugt, dass es zur Auslösung des automatischen Zurückbewegens in die Schutzposition vorgängig einer Betätigung eines Auslöseorgans mit der das Gehäuse haltenden Hand bedarf, also ohne das Gehäuse mit der daran gehaltenen Einstecheinrichtung vorher abzulegen oder die zweite Hand zur Hilfe zu nehmen. Hierbei ist es beispielsweise bevorzugt, wenn ein Entriegelungsknopf vorhanden ist, welcher mit einem freien Finger der das Gehäuse haltenden Hand gedrückt werden kann, oder wenn ein erneutes Bewegen der beiden Gehäuseteile bzw. Griffkomponenten relativ zueinander in eine Richtung, in welcher diese bereits zueinander bewegt wurden, um die Bewegung der Einführ- und Einstecheinrichtung von der Schutz- in die Einführposition zu bewirken, die Auslösung der automatischen Rückstellung in die Schutzposition bewirkt.

Ein solcher erfindungsgemässer Insertionskopf ist denkbar einfach und sicher zu bedienen.

Der Insertionskopf ist für eine medizinische oder pharmazeutische, einschließlich kosmetische, Anwendung vorgesehen. Zumindest die Unterseite der Basis ist gewebeverträglich gebildet. Der Insertionskopf ist vorzugsweise Bestandteil eines Infusionssets für die Verabreichung von Insulin, eines Schmerzmittels oder eines anderen per Infusion verabreichbaren Medikaments. Anstatt für eine Medikamentenverabreichung oder grundsätzlich auch eines anderen verabreichbaren Produkts kann der Insertionskopf auch zu Diagnosezwecken dienen. In solchen Applikationen kann die Einführeinrichtung Träger eines Sensors zum Messen von beispielsweise der Glukosekonzentration in einer Körperflüssigkeit oder einer anderen physikalischen und/oder biochemischen Größe dienen, die für den Gesundheitszustand eines Patienten maßgeblich ist oder sein kann. Der Insertionskopf kann zu Diagnosezwecken auch als Perfusionsvorrichtung gebildet sein. In solch einer Ausbildung wird die Einführeinrichtung nach dem Einbringen in das Gewebe von einer Spülflüssigkeit durchströmt, die ein, oder mehrere bestimmte Inhaltsstoffe der Körperflüssigkeit bei dem Durchströmen aufnimmt, um die mit dem betreffenden Inhaltsstoff oder den mehreren Inhaltsstoffen angereicherte Spülflüssigkeit zu analysieren. Schließlich kann der Insertionskopf eine Vorrichtung für die Verabreichung eines Produkts und eine Diagnoseeinrichtung in Kombination bilden. Die Einführeinrichtung kann für die Zuführung eines Produkts, das insbesondere ein Medikament oder eine Spülflüssigkeit sein kann, oder die Abführung einer Körperflüssigkeit oder nur eines oder mehrerer bestimmter Inhaltsstoffe einer Körperflüssigkeit gebildet sein, d.h. die Einführeinrichtung bildet in solch einer Applikation wenigstens einen Strömungsquerschnitt. Die Einführeinrichtung kann der Zu- und Abführung von Stoffen auch in Kombination dienen. Ist der Insertionskopf nur als Messvorrichtung gebildet, so kann er auch nur dazu dienen, einen Sensor oder einen Teil eines Sensors zu platzieren, d.h. rein als mechanische Einbringeinrichtung. In einer Weiterbildung als Messvorrichtung kann er über das mechanische Einbringen hinaus auch der Übertragung von Steuersignalen zu dem Sensor und/oder von Messsignalen von dem Sensor dienen. In kombinierten Applikationen kann er schließlich über wenigstens einen Strömungsquerschnitt für den Stofftransport, d.h. eine Strömungsleitung, und wenigstens eine Signalleitung verfügen. Auf die Signalleitung kann verzichtet werden, wenn der Sensor für den drahtlosen Empfang von Steuersignalen und/oder das drahtlose Senden von Messsignalen eingerichtet ist. Schließlich kann die Einführeinrichtung auch zwei oder mehrere Einführeinrichtungen mit zugeordneten Einstecheinrichtungen aufweisen, die separat abragen. So kann eine erste Einführeinrichtung dem Stofftransport in das Gewebe und eine andere dem Stofftransport aus dem Gewebe oder nur dem Einbringen eines Sensors oder eines Teils eines Sensors dienen. Mit mehreren Einführeinrichtungen, die je einen Strömungsabschnitt aufweisen, können mit dem gleichen Insertionskopf auch unterschiedliche Stoffe verabreicht werden. Dies kann auch mit einer Einführeinrichtung verwirklicht werden, die mehrere, separate Strömungsquerschnitte in einem gemeinsamen Abschnitt bildet.

In einer bevorzugten Ausführungsform des Insertionskopfes sind die beiden Gehäuseteile zwischen zwei Fingern einer Hand greifbar, bilden also eine erste und eine zweite Griffkomponente, wobei mit je einem der Finger gegen eines der Gehäuseteile drückend das zweite Gehäuseteil in Richtung auf das erste Gehäuseteil zu bewegbar ist, zur Bewirkung der Bewegung der Einsetz- und Einstecheinrichtung von der Schutzposition in die Einsetzposition. Hierdurch wird es möglich, den erfindungsgemässen Insertionskopf ohne weitere Hilfsmittel, wie z.B. eine speziell darauf angepasste Insertionsvorrichtung, einfach und sicher zu verwenden.

Bevorzugterweise ist ein erstes der beiden relativ zueinander beweglichen Gehäuseteile nicht beweglich mit der Basis verbunden. Grundsätzlich ist es jedoch auch vorgesehen, dass beide der relativ zueinander beweglichen Gehäuseteile relativ zu der Basis beweglich sind und insbesondere relativ zu der Basis beweglich mit dieser verbunden sind.

Für die Übertragung der Relativbewegung der beiden zueinander beweglichen Gehäuseteile auf die Einführeinrichtung und die Einstecheinrichtung kann eine steife Kopplung vorgesehen sein, d.h. eines der beiden Gehäuseteile und die Einführstecheinrichtung können steif miteinander verbunden sein, worunter auch eine Urformung in einem Stück verstanden wird. Eine steife Kopplung kann beispielsweise im Falle einer bevorzugten Schwenkbeweglichkeit der Einführ- und Einstecheinrichtung gegenüber der Basis ohne Weiteres dann verwirklicht werden, wenn auch das steif mit der Einstecheinrichtung ausgebildete Gehäusesteil schwenkbeweglich gegenüber der Basis ist. Gegenüber dem Insertionskopf der deutschen Patentanmeldung Nr. 10 2004 039 408.3 weist der erfindungsgemäße Insertionskopf den Vorteil auf, dass das andere der beiden relativ zueinander beweglichen Gehäuseteile.dem Benutzer als Widerlager dienen kann und nicht das Gewebe über die Basis die für die Schwenkbewegung aufzubringende Kraft aufnehmen muss. Verfügt der Benutzer über eine geeignete Insertionsvorrichtung, auch Inserter genannt, mit welcher der Insertionskopf auf dem Gewebe platziert und dabei die Einführeinrichtung ins Gewebe eingeführt wird, wird diese Kraft innerhalb der Insertionsvorrichtung aufgenommen.

In einer weiteren bevorzugten Ausführungsform des Insertionskopfes ist die Basis unbeweglich mit der Einführeinrichtung verbunden, so dass sie beim Bewegen derselben von der Schutzposition in die Einführposition mit dieser mitbewegt wird. Hierdurch lässt sich die Verbindung zwischen Einführeinrichtung und Basis denkbar einfach und daher kostengünstig gestalten.

In einer alternativen Ausführungsform zu der zuvor dargelegten bildet die Unterseite der Basis bei in der Schutzposition befindlicher Einführ- und Einstecheinrichtung zumindest einen Teil einer Aussenseite des Gehäuses, bevorzugterweise der Unterseite des Gehäuses, so dass deren freie Enden in der Schutzposition hinter der Unterseite der Basis zurückstehen. Dabei sind die Einführeinrichtung und die Einstecheinrichtung durch Bewegen derselben von der Schutzposition in die Einführposition gegenüber der Basis bewegbar, derart, dass deren freie Enden in der Einführposition über die Unterseite der Basis vorragen. Solche Ausführungsformen erlauben es, die Basis als Unterseite des Gehäuses auszubilden, wodurch besonders kompakte erfindungsgemässe Insertionsköpfe möglich werden. Dabei ist es bei Ausführungsformen, bei denen das Gehäuse einen Griff bildet, wobei die beiden Gehäuseteile eine erste und eine zweite Griffkomponente bilden, bevorzugt, wenn dieser Griff von der Basis abragt, wobei die erste Griffkomponente gegenüber der Basis unbeweglich ist und die zweite Griffkomponente relativ zu der ersten Griffkomponente und der Basis beweglich ist, bevorzugterweise verschiebbar oder verschwenkbar ist. Die bewegliche zweite Griffkomponente ist dabei mit der Einführeinrichtung so gekoppelt, dass eine Bewegung der zweiten Griffkomponente eine Bewegung der Einführeinrichtung in die Einführposition bewirkt. Aufgrund der Ausstattung des Griffs mit einer beweglichen Griffkomponente kann die Bewegung der Einführeinrichtung allein durch Greifen und Betätigen des Griffs bewirkt werden. Der Griff bildet selbst das Widerlager für die bewegbare zweite Griffkomponente. In diesem Sinne wird hier der das Widerlager bildende Teil des Griffs als erste Griffkomponente bezeichnet. Die zweite Griffkomponente kann beispielsweise als Druckknopf gebildet sein. Die erste Griffkomponente kann ein Gehäuse sein, aus dem solch ein Druckknopf herausragt. In ebenfalls bevorzugten Ausführungen bilden die beiden Griffkomponenten erst gemeinsam den Griff, beispielsweise die Hälften eines insgesamt zweiteiligen Griffs.

In noch einer weiteren bevorzugten Ausführungsform des Insertionskopfes ist die Einführeinrichtung in der Basis beweglich gelagert, z.B. verschiebbar und/oder schwenkbar. Hierdurch ergibt sich der Vorteil, dass diese Lagerung gleichzeitig die Verbindung zwischen Basis und Einführeinrichtung im applizierten Zustand bilden kann, so dass auf zusätzliche Verbindungselemente verzichtet werden kann.

Dabei ist es bevorzugt, wenn die Basis und ein Gelenkelement oder Führungselement einer Schiebeführung miteinander ein Gelenk oder eine lineare oder nichtlineare Schiebeführung bilden und die Einführeinrichtung von dem Gelenkelement oder dem Führungselement abragt. Ein solches Gelenk- oder Führungselement könnte z.B. gleichzeitig als Kanülengehäuse dienen und einen Flüssigkeitspfad zur Zuführung oder Abführung von Flüssigkeit von der Einführeinrichtung beinhalten.

Auch ist es bei Ausführungsformen des Insertionskopfes, bei denen die Unterseite der Basis bei in der Schutzposition befindlicher Einführ- und Einstecheinrichtung zumindest einen Teil einer Aussenseite des Gehäuses bildet, bevorzugt, dass das Gehäuse von einer Oberseite der Basis aufragt, also vollständig oberhalb der Basis angeordnet ist.

In noch einer weiteren bevorzugten Ausführungsform des erfindungsgemässen Insertionskopfes sind die beiden Gehäuseteile bzw. die erste und die zweite Griffkomponente relativ zueinander schwenkbar oder verschiebbar, bevorzugterweise ineinander schiebbar sind, zur Bewirkung der Bewegung der Einführeinrichtung und der Einstecheinrichtung von der Schutzposition in die Einführposition.

Dabei ist es bevorzugt, dass die beiden Gehäuseteile bzw. Griffkomponenten zur Bewirkung dieser Bewegung linear zueinander verschiebbar sind.

Dabei ist es bei Ausführungsformen, bei denen die Unterseite der Basis bei in der Schutzposition befindlicher Einführ- und Einstecheinrichtung zumindest einen Teil einer Aussenseite bzw. der Unterseite des Gehäuses bildet, weiter bevorzugt, dass die beiden Gehäuseteile im Wesentlichen parallel zur Unterseite der Basis bewegbar sind. Insbesondere kann es sich um eine Linearbeweglichkeit handeln.

Solche Ausführungsformen erleichtern die einhändige Bedienung der erfindungsgemässen Insertionskopfes deutlich und ermöglichen die Bildung besonders kompakter erfindungsgemässer Insertionsköpfe.

In noch einer weiteren bevorzugten Ausführungsform des erfindungsgemässen Insertionskopfes ist die Einführeinrichtung zusammen mit der Einstecheinrichtung durch das Bewegen der beiden Gehäuseteile bzw. Griffkomponenten relativ zueinander von der Schutzposition in die Einführposition schwenkbar. Eine Schwenkbeweglichkeit lässt sich auf einfache und kostengünstige Weise bewerkstelligen, ermöglicht die Bereitstellung kompakter erfindungsgemässer Insertionsköpfe und ist zudem funktionssicher. Dabei ist es bevorzugt, dass in dem Moment, wenn die Einführeinrichtung bzw. die Einstecheinrichtung mit ihrem freien Ende über die Begrenzungen des Gehäuses bzw. die Unterseite des Gehäuses oder der Basis vorschwenkt, die Längsachse der Einführeinrichtung mit der Unterseite der Basis einen spitzen Winkel von vorzugsweise weniger als 50° einschließt. Vorzugsweise ist der Winkel kleiner als 30°, so dass die Längsachse bzw. die Einführeinrichtung im Moment des Ausschwenkens zumindest im Wesentlichen parallel zu der Begrenzung bzw. Unterseite des Gehäuses oder der Auflagefläche der Basis ist.

Auch ist es bei Ausführungsformen des Insertionskopfes, bei denen die Einführeinrichtung und die Einstecheinrichtung um eine Rotationsachse von der Schutzposition in die Einführposition geschwenkt werden, bevorzugt, dass die Längsachse der Einführeinrichtung, die um die Rotationsachse geschwenkt wird, die Rotationsachse schneidet. Falls die Längsachse der Einführeinrichtung die Rotationsachse nicht schneidet, sondern in einem Abstand kreuzt, ist der Abstand vorzugsweise deutlich kleiner als die Länge der Einführeinrichtung. Vorzugsweise ist der Abstand höchstens halb so groß wie die bestimmungsgemässe Eindringtiefe bzw. Länge der Einführeinrichtung. Der Schwenkwinkel der Einführeinrichtung beträgt in bevorzugten Ausführungen 90° ± 10°. In ebenfalls vorteilhaften Ausführungen kann der Schwenkwinkel jedoch auch kleiner sein, insbesondere falls die Einführeinrichtung auch gegenüber der Basis geschwenkt wird und in der Einführposition nicht rechtwinklig zur Unterseite der Basis weist, sondern in einem spitzen Winkel, der allerdings wenigstens 30° betragen sollte. Entsprechend beträgt der Schwenkwinkel in derartigen Ausführungen vorzugsweise wenigstens etwa 30° oder jeden Zwischenwert zwischen etwa 30° und etwa 90°. Grundsätzlich kann der Schwenkwinkel auch größer als 90° sein.

In bevorzugten Ausführungsformen des erfindungsgemässen Insertionskopfes umfasst die Kopplung, welche die Relativbewegung der beiden Gehäuseteile bzw. Griffkomponenten zueinander in die Bewegung der Einführeinrichtung und der Einstecheinrichtung überträgt, ein Getriebe. Eine derartige Kopplung hat den Vorteil, dass die Beweglichkeit der Gehäuseteile zueinander nicht der Beweglichkeit der Einführeinrichtung entsprechen muss, sondern beide Beweglichkeiten jeweils einzeln für sich optimal gestaltet werden können. So kann die Einführeinrichtung insbesondere schwenkbeweglich und können die Gehäuseteile translatorisch zueinander beweglich und dabei vorzugsweise linear geführt sein. Im Falle einer Schwenkbeweglichkeit eines der Gehäuseteile kann dessen Schwenkachse eine andere als die der Einführeinrichtung sein. Während die Einführeinrichtung vorzugsweise um eine zu einer Begrenzung oder der Unterseite des Gehäuses oder der Basis zumindest im Wesentlichen parallelen Rotationsachse schwenkbar ist, was im übrigen für sämtliche Ausführungsformen des erfindungsgemässen Insertionskopfes mit schwenkbarer Einführeinrichtung gilt, kann ein schwenkbares Gehäuseteil um eine zu dieser Begrenzung oder Unterseite des Gehäuses oder der Basis zumindest im Wesentlichen rechtwinkligen Rotationsachse schwenkbeweglich sein. Ein Getriebe kann aber auch dann von Vorteil sein, wenn im Falle eines schwenkbeweglichen Gehäuseteils dessen Rotationsachse von der Rotationsachse der schwenkbeweglichen Einführeinrichtung parallel beabstandet ist. In solch einem Fall kann der Schwenkwinkel des Gehäuseteils mittels eines Getriebes untersetzt oder vorzugsweise übersetzt auf die Einführeinrichtung übertragen werden.

Eine bevorzugte Ausführungsform des erfindungsgemässen Insertionskopfes mit einer getriebetechnischen Kopplung umfasst ein Zahnrad und eine Zahnstange, die miteinander in einem Zahneingriff sind und bei Bewegung der beiden Gehäuseteile relativ zueinander miteinander kämmen, unter Bewegung der Einführeinrichtung. Die Zahnstange ist vorzugsweise mit einem der beiden Gehäuseteile verbunden, so dass eine Bewegung dieses Gehäuseteils gegenüber der Drehachse des Zahnrads in Längsrichtung der Zahnstange in eine Drehbewegung des in diesem Fall mit der Einführeinrichtung verbundenen Zahnrads übertragen wird. Bei sehr kleinem Zahnraddurchmesser und entsprechend feiner Verzahnung bzw. Zahnteilung kann ein vergleichsweise kurzer Hub der Gehäuseteile relativ zueinander in eine Drehbewegung des Zahnrads über einen beträchtlichen Teil einer vollen Umdrehung, vorzugsweise in eine viertel Umdrehung des Zahnrads, übertragen werden. Vorteilhafterweise ist die bewegliche zweite Griffkomponente mit der Zahnstange in einem Stück geformt. Auch ist es bevorzugt, dass die Einführeinrichtung drehsteif mit dem Zahnrad verbunden ist.

In noch einer weiteren bevorzugten Ausführungsform des erfindungsgemässen Insertionskopfes ist es vorgesehen, dass die Einführeinrichtung und/oder die Einstecheinrichtung über eine Dreh- oder Schwenkachse an der Basis oder an einem ersten der beiden Gehäuseteile gehalten ist. Ein Kulissenstein ist vorhanden, der in Bezug auf die Drehachse exzentrisch angeordnet ist und bevorzugt getrennt von dieser angeordnet ist.

Weiter ist eine Kulissenführung vorhanden, die im Wirkzusammenhang mit dem Kulissenstein steht, so dass eine Bewegung der beiden Gehäuseteile relativ zueinander den Kulissenstein geführt durch die Kulissenführung aus einer ersten Stellung, die der Schutzposition der Einführeinrichtung bzw. Einstecheinrichtung entspricht, in eine zweite Stellung, die der Einführposition der Einführeinrichtung und der Einstecheinrichtung entspricht, überführt. Diese Ausbildung ermöglicht relativ komplexe Bewegungsabläufe, wobei gleichzeitig relativ geringe Kräfte über die Gehäuseteile ausgeübt werden müssen und ein Verkanten zwischen den einzelnen Bestandteilen der Kopplungsmechanik höchst unwahrscheinlich ist.

In noch einer weiteren bevorzugten Ausführungsform ist das Gehäuse lösbar mit der Basis verbunden. Bevorzugterweise löst sich diese Verbindung automatisch bei der Bewegung der beiden Gehäuseteile relativ zueinander, und zwar bevorzugterweise bei der Bewegung der beiden Gehäuseteile zueinander, welche die Bewegung der Einführeinrichtung von der Schutz- in die Einführposition bewirkt oder bei einer insbesondere direkt darauf folgenden bzw. weiterführenden Bewegung der beiden Gehäuseteile relativ zueinander. Alternativ wäre es grundsätzlich jedoch ebenfalls denkbar, das Gehäuse oder die Basis mit einer weiteren beweglichen Komponente auszustatten, durch deren Betätigung die Verbindung mit der Basis gelöst wird. Die Verbindung zwischen dem Gehäuse und der Basis kann zwar durch einen reinen Reibschluss hergestellt sein, bevorzugter beruht sie jedoch ausschließlich auf einem Formschluss oder einer Kombination aus Form- und Reibschluss. Um die Verbindung zu schaffen, sind die Basis und das Gehäuse, vorzugsweise eines der Gehäuseteile, je mit wenigstens einem Verbindungselement ausgestattet, die bei bestehender Verbindung miteinander in einem Eingriff sind. Um die Verbindung lösen zu können, ist vorzugsweise wenigstens eines der Verbindungselemente gegen eine rückstellende Elastizitätskraft aus dem Eingriff bewegbar. In bevorzugten Ausführungen dient eines der beiden Gehäuseteile, welches gegenüber der Basis bewegbar ist, nicht nur der Überführung der Einführeinrichtung in die Einführposition, sondern auch dem Lösen der Verbindung, indem dieses Gehäuseteil bei seiner Bewegung durch Kontakt, vorzugsweise Gleitkontakt, eines der Verbindungselemente gegen die Elastizitätskraft aus dem Eingriff bewegt, beispielsweise elastisch abbiegt

Auch ist es bei Ausführungsformen des erfindungsgemässen Insertionskopfes mit einer Kopplung mit Kulissenführung und einem lösbar mit der Basis verbundenem Gehäuse bevorzugt, dass der Insertionskopf derartig ausgestaltet ist, dass eine Bewegung der beiden Gehäuseteile relativ zueinander über eine erste Strecke eines ersten Abschnitts der Kulissenführung den Kulissenstein, geführt durch die Kulissenführung, aus einer ersten Stellung, die der Schutzposition der Einführeinrichtung und der Einstecheinrichtung entspricht, in eine zweite Stellung, die der Einführposition der Einführeinrichtung und der Einstecheinrichtung entspricht, überführt. Weiter ist ein Verbindungselement vorhanden, welches die Basis reversibel mit dem Gehäuse verbindet, wobei zum Lösen des Gehäuses von der Basis bevorzugterweise genau eines der beiden Gehäuseteile über eine zweite Strecke senkrecht zur Erstreckungsrichtung der in der Einführposition befindlichen Einführeinrichtung gegenüber der Basis bewegbar ist, wobei die Kulissenführung einen zu der zweiten Strecke korrespondierenden zweiten Abschnitt aufweist, so dass die Einführeinrichtung beim Lösen nicht mitbewegt wird. D.h., durch die passende Ausbildung der Kulissenführung ist es z.B. möglich, nach dem Einstechen der Einstecheinrichtung und der Einführeinrichtung in den Körper eines Patienten eine Bewegung des Gehäuses oder eines der Gehäuseteile parallel zur Körperoberfläche bzw. zur Oberfläche eines die Basis auf dem Körper des Patienten befestigenden Pflasters zu bewerkstelligen, ohne dass dabei das organische Gewebe strapaziert wird. Auf diese Weise ist eine Trennung des Gehäuses von der Basis und damit der Einstecheinrichtung von der Einführeinrichtung in vorteilhafter Weise durch Betätigen derselben Komponenten möglich, und hier insbesondere der beiden Gehäuseteile relativ zueinander, um sowohl die Bewegung der Einführeinrichtung und der Einstecheinrichtung in die Einführposition als auch die Entkopplung des Gehäuses von der Basis zu bewirken. Es ist auch möglich, die Bewegung und Entkopplung unmittelbar hintereinander durchzuführen. Dabei durchläuft der Kulissenstein bereits die gesamte Kulissenführung, wodurch die Kanüle bzw. Einführeinrichtung in Verbindung mit der Einstichnadel bzw. Einstecheinrichtung bereits ausgeschwenkt wird, und gleichzeitig oder unmittelbar anschließend die Entkopplung des Gehäuses von der Basis durchgeführt wird, bevor die Kanüle mit der Nadel appliziert wird. Hier besteht dann nur noch ein leicht aufhebbarer Zusammenhalt zwischen dem im bzw. am Körper des Patienten verbleibenden Abschnitt (Basis, Kanüle) und dem zu entfernenden Abschnitt (Einstichnadel, Gehäuse) des erfindungsgemäßen Insertionskopfes.

In noch einer weiteren bevorzugten Ausführungsform des Insertionskopfes ist die Einstecheinrichtung in der Schutzposition noch nicht mit dem Gehäuse verbunden, verbindet sich jedoch bei der Bewegung in die Einführposition vorzugsweise automatisch mit dem Gehäuse. Hierfür kann an der Einführeinrichtung an dem von ihrem freien Ende abgewandten Ende ein Verbindungselement, vorzugsweise ein Schnappelement, vorgesehen sein, das zugleich mit dem Abschluss der Bewegung in die Einführposition oder kurz zuvor mit einem Verbindungsgegenelement des Gehäuses in einen Verbindungseingriff gelangt. Die Verbindung kann zwar grundsätzlich rein reibschlüssig sein, bevorzugterweise umfasst sie jedoch zumindest einen Formschluss. Das Verbindungselement der Einstecheinrichtung kann mit dem Verbindungsgegenelement insbesondere eine Schnappverbindung bilden. Grundsätzlich genügt für eine formschlüssige Verbindung auch nur ein einfacher Hintergriff bezüglich der Richtung, in die das Gehäuse von der Einführeinrichtung bzw. von der Basis entfernt werden soll; ein elastischer Schnappeingriff ist daher nicht zwangsläufig erforderlich.

So ist es z.B. bei der zuvor erwähnten Ausführungsform bevorzugt, dass eines der beiden Gehäuseteile soweit in das andere Gehäuseteil eingeschoben werden kann, dass eine Steuerrampe an einem der beiden Gehäuseteile nach Beendigung des Vorgangs zur Überführung der Einführeinrichtung von der Schutzposition in die Einführposition gegen eine Auslenkrampe an einem Verbindungselement, das die Basis reversibel mit dem Gehäuse verbindet, anlaufen kann, um eine Verbindungseinrichtung aus ihrer Eingriffsstellung auszulenken, so dass der Griff und die Basis trennbar sind. Diese Ausbildung insbesondere in Verbindung mit der zweiten Strecke einer Kulissenführung wie bereits zuvor beschrieben ist besonders vorteilhaft, um ohne Beanspruchung des Gewebes des Patienten den erfindungsgemäßen Insertionskopf von der Einstechfunktion in die Zuführfunktion zum Zuführen eines Medikaments zu überführen.

In noch einer weiteren bevorzugten Ausführungsform des Insertionskopfes sind in der Schutzposition zumindest die freien Enden der Einführeinrichtung und der Einstecheinrichtung, bevorzugterweise die gesamte Einführeinrichtung und die gesamte Einstecheinrichtung, in einer Aufnahme aufgenommen, welche entweder von der Basis oder dem Gehäuse gebildet ist. Falls das Gehäuse die Aufnahme bildet, kann die Basis eine Teilaufnahme bilden, die in der vom Gehäuse gebildeten Aufnahme aufgenommen ist, solange im Falle des bevorzugt lösbaren Gehäuses dieses mit der Basis verbunden ist.

In noch einer weiteren bevorzugten Ausführungsform des Insertionskopfes ist der Einführeinrichtung eine Sicherungsstruktur zugeordnet, welche die Einführeinrichtung bzw. die Einstecheinrichtung in der Schutzposition reversibel zurückhält. So kann beispielsweise eines der beiden Gehäuseteile mit einer Rastschulter ausgebildet sein, die in einen reversiblen Eingriff mit einem komplementären Rastorgan am anderen Gehäuseteil bringbar ist. Wird ein Widerstand überwunden, kann eine Relativbewegung der beiden Gehäuseteile zueinander erfolgen und die Einführeinrichtung (Kanüle) nebst Einstecheinrichtung (Einstechnadel) kann in die Einführposition (Applikationsstellung) verfahren oder geschwenkt werden.

Dabei kann in vorteilhafter Weise der Einführeinrichtung ein Kanülengehäuse zugeordnet sein, das mit der Einführeinrichtung mitbewegt wird, wobei das Kanülengehäuse die Sicherungsstruktur umfasst. Diese sollte einer parallel zu der Bewegungsrichtung der beiden Gehäuseteile zueinander erstreckten Wand eines der beiden Gehäuseteile gegenüberliegen. Dadurch wird es einem Eingriffselement an der Innenseite der Wand mit der Sicherungsstruktur ermöglicht, in der Schutzposition für die Einführeinrichtung im reversiblen Eingriff zu stehen. Mittels dieser Maßnahmen ist es möglich, die Einführeinrichtung zusammen mit der Einstecheinrichtung sicher in der Schutzposition in der Aufnahme zu halten, um anschliessend nach Überwindung eines anfänglichen Widerstandes, der durch die Sicherungsstruktur und das Eingriffselement bereitgestellt wird, durch Bewegung der beiden Gehäuseteile relativ zueinander die gewünschte Bewegung der Einführeinrichtung in Verbindung mit der Einstecheinrichtung von der Schutzposition in die Einführposition bewerkstelligen zu können.

In noch einer weiteren bevorzugten Ausführungsform des erfindungsgemässen Insertionskopfs ist in einem der beiden Gehäuseteile und/oder in dem anderen der beiden Gehäuseteile, welches die Einführeinrichtung nebst Einstecheinrichtung in der Schutzposition aufnimmt, eine Sicherungskulisse vorhanden, in der die Einstecheinrichtung nach dem Entfernen des Gehäuses und der Einstecheinrichtung von der Einführeinrichtung und der Basis und dem Zurückbewegen in die Schutzposition in einer Aufnahme einrastbar ist. Durch ihre federnde Eigenschaft kann die Spitze der Einstecheinrichtung entlang der Sicherungskulisse beim Zurückschwenken in die Schutzposition bzw. in die Aufnahme elastisch verformt bzw. seitlich ausgelenkt werden, um anschließend, bevorzugterweise irreversibel, in eine Sicherungsausnehmung hinter einer Sicherungsschulter einzurasten. In dieser Position wird sie dann sicher in der Schutzposition gehalten. Die Sicherungskulisse kann auch an verschiedenen anderen Bestandteilen des Insertionskopfes vorgesehen sein, welche beim Entfernen der Einstecheinrichtung von der Einführeinrichtung und der Basis mit entfernt werden.

In noch einer weiteren bevorzugten Ausführungsform des Insertionskopfes ist der Einstecheinrichtung ein Halteelement zugeordnet, das bei dem automatischen Zurückbewegen der Einstecheinrichtung in eine Schutzposition mit der Einstecheinrichtung mitbewegt wird, wobei das Halteelement eine Rastschulter aufweist, die bei dem automatischen Zurückbewegen im Gehäuse in einen verrastenden Eingriff mit einem Eingriffelement tritt, um ein neuerliches Herausbewegen der Einstecheinrichtung aus dem Gehäuse zu verhindern.

In noch einer weiteren bevorzugten Ausführungsform des Insertionskopfes ist dieser ausgestaltet für die automatisierte Platzierung auf dem Gewebe mittels einer Insertionsvorrichtung, auch Inserter genannt. Hierzu weist der Insertionskopf, vorzugsweise dessen Gehäuse, eine Haltestruktur auf, die mit der Halteeinrichtung des Inserters in einen Halteeingriff gebracht werden kann. Falls, was bevorzugt ist, eines der beiden relativ zueinander beweglichen Gehäuseteile des Insertionskopfes unbeweglich mit der Basis verbunden ist und das andere der beiden Gehäuseteile relativ zur Basis beweglich ist, bildet vorzugsweise das unbewegliche Gehäuseteil die Haltestruktur. Die Haltestruktur ist bevorzugterweise an dem Gehäuse oder dem gegenüber der Basis unbeweglichen Gehäuseteil insbesondere in einem Stück angeformt, vorzugsweise ist sie mit dem Gehäuse bzw. dem Gehäuseteil zumindest steif verbunden.

Weitere bevorzugte Merkmale werden in den abhängigen Patentansprüchen und deren Kombinationen offenbart.

Nachfolgend werden Ausführungsbeispiele der Erfindung anhand von Figuren erläutert. An den Ausführungsbeispielen offenbar werdende Merkmale bilden je einzeln und in jeder technisch sinnvollen Merkmalskombination die Gegenstände der Ansprüche und auch die vorstehend beschriebenen Ausgestaltungen vorteilhaft weiter. Es zeigen:
Fig. 1 einen Vertikalschnitt durch eine erste Ausführungsform eines erfindungsgemässen Insertionskopfes mit in Schutzposition angeordneter Einführ- und Einstecheinrichtung;
Fig. 2 einen Längsschnitt durch den Insertionskopf aus Fig. 1 mit in der Einführposition angeordneter Einführ- und Einstecheinrichtung;
Fig. 3 einen Längsschnitt durch den Insertionskopf aus Fig. 1 nach der Applikation beim Entfernen der Einstecheinrichtung;
Fig. 4 eine Darstellung wie Fig. 3, wobei die Einstecheinrichtung wieder in der Schutzposition angeordnet ist;
Fig. 5 einen oberen Abschnitt eines ersten Gehäuseteils eines zweiten erfindungsgemässen Insertionskopfes im Horizontalschnitt;
Fig. 6 einen in den ersten Gehäuseteil, dessen oberer Abschnitt in Fig. 5 dargestellt ist, einschiebbaren zweiten Gehäuseteil in der Seitenansicht;
Fig. 7 eine Ansicht von unten auf die beiden in einer Grundposition zueinander angeordneten Gehäuseteile aus den Figuren 5 und 6;
Fig. 8 eine Ansicht von unten auf die beiden Gehäuseteile aus den Figuren 5 und 6 in einer zusammen geschobenen und miteinander verrasteten Situation;
Fig. 9 eine Ansicht von unten auf die beiden durch erneutes Zusammendrücken und anschliessendes Entlasten wieder in der Grundposition gemäss Fig. 5 zueinander angeordneten Gehäuseteile aus den Figuren 5 und 6;
Fig. 10 ein System aus einem Insertionskopf und einem Inserter eines ersten Ausführungsbeispiels vor einer Aktivierung;
Fig. 11 das System des ersten Ausführungsbeispiels nach der Aktivierung;
Fig. 12 ein System aus einem Insertionskopf und einem Inserter eines zweiten Ausführungsbeispiels vor einer Aktivierung des Insertionskopfs;
Fig. 13 das System des zweiten Ausführungsbeispiels nach der Aktivierung;
Fig. 14 das System des zweiten Ausführungsbeispiels nach einer Platzierung des Insertionskopfs auf einer Gewebeoberfläche;
Fig. 15 einen Insertionskopf mit in Schutzposition befindlicher Einführeinrichtung in einer Seitenansicht;
Fig. 16 den Insertionskopf nach Fig. 15 mit in Einführposition befindlicher Einführeinrichtung;
Fig. 17 den Insertionskopf nach den Fig. 15 und 16, wobei der Griff nebst Einstechnadel von der Basis nebst Einführeinrichtung getrennt ist;
Fig. 18 die erste und die zweite Griffkomponente im von der Basis gelösten Zustand in einer Seitenansicht;
Fig. 19 einen Insertionskopf mit in Schutzposition befindlicher Einführeinrichtung in einer Schnittansicht;
Fig. 19a einen Ausschnitt aus Fig. 19 ebenfalls in einer Schnittdarstellung;
Fig. 20 den Insertionskopf gemäß Fig. 19 mit in Einführposition befindlicher Einführeinrichtung, ebenfalls in einer Schnittdarstellung;
Fig. 21 in einer Schnittdarstellung den Griff nebst Einstichabschnitt getrennt von der Basis mit Einführeinrichtung, die am Patienten platziert sein kann;
Fig. 22 in einer Schnittdarstellung die erste und die zweite Griffkomponente nach dem Herausschieben der zweiten Griffkomponente aus der ersten Griffkomponente, nebst einem vergrößerten Ausschnitt C ebenfalls in Schnittdarstellung;
Fig. 22a einen Schnitt D-D gemäß Fig. 22, der senkrecht zu dem Schnitt gemäß Fig. 22 angelegt ist;
Fig. 23 einen Insertionskopf gemäß der Erfindung in einer perspektivischen Ansicht vor der Applikation;
Fig. 24 den Insertionskopf gemäß Fig. 23 nach der Applikation;
Fig. 25 den Insertionskopf gemäß den Fig. 23 und 24 nach Entfernung der ersten und zweiten Griffkomponenten nebst Einstechnadel;
Fig. 26 die erste und zweite Griffkomponente in einer perspektivischen Ansicht nach dem Herausziehen der zweiten Griffkomponente aus der ersten Griffkomponente;
Fig. 27 die Basis des Insertionskopfes nach den Fig. 23 bis 26 nach dem Andocken eines Konnektors zur Zuführung eines Medikaments;
Fig. 28 einen Insertionskopf mit in Schutzposition befindlicher Einführeinrichtung nebst Einstechnadel, dargestellt ohne erste Griffkomponente;
Fig. 29 den Insertionskopf gemäß Fig. 28 mit Einführeinrichtung nebst Einstechnadel in Applikationsposition;
Fig. 30 einen Insertionskopf mit in Schutzposition befindlicher Einführeinrichtung in einer Seitenansicht; und
Fig. 31 einen Schnitt E-E durch den Insertionskopf gemäß Fig. 30 in einer Draufsicht.

Nachfolgend werden gleiche oder zumindest funktionsgleiche Bestandteile in der Regel mit gleichen oder vergleichbaren Bezugszeichen benannt, so dass eine mehrfache Beschreibung meist unterbleiben kann. Die Bestandteile der einzelnen Ausführungsformen können grösstenteils miteinander ausgetauscht werden, d.h. kombiniert werden. Aus den nachfolgend beschriebenen Ausführungen gehen weitere Merkmale, Zielsetzungen sowie Vorteile der Erfindung hervor.

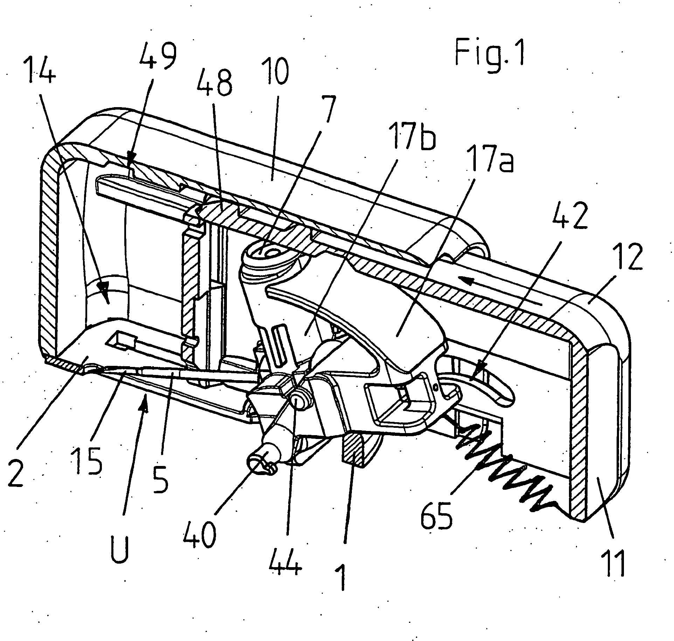

Fig. 1 zeigt eine erste bevorzugte Ausführungsform eines erfindungsgemässen Insertionskopfs in einem Längsschnitt. Der Insertionskopf umfasst eine Basis 1, 2 die in einem Stück aus Kunststoff geformt ist. Die Basis 1, 2 ist mit ihrer Unterseite U auf organischem Gewebe platzierbar. Der Insertionskopf umfasst des Weiteren ein Gehäuse 10, 12 aus zwei relativ zueinander bewegbaren Gehäuseteilen 10, 12, welche einen zweiteiligen Griff mit einer ersten Griffkomponente 10 und einer zweiten Griffkomponente 12 bilden. Die erste Griffkomponente 10 ist mit der Basis 1, 2 unbeweglich, aber lösbar verbunden. Die zweite Griffkomponente 12 ist an der ersten Griffkomponente 10 beweglich gehalten, wobei die zweite Griffkomponente 12 sowohl relativ zu der ersten Griffkomponente 10 als auch zu der Basis 1, 2 linear verschiebbar ist. Die Achse der Bewegbarkeit der zweiten Griffkomponente 12 weist parallel zu einer Unterseite U der Basis 1, 2. Die Richtung der Bewegbarkeit ist auf der Oberseite der zweiten Griffkomponente 12 mit einem Pfeil angedeutet.

In der ersten Griffkomponente 10 ist eine Einführeinrichtung 5 schwenkbeweglich gegenüber der Basis 1, 2 um eine zur Unterseite U der Basis 1, 2 parallele Rotationsachse gelagert. Die Einführeinrichtung 5 ist lang gestreckt. Im Ausführungsbeispiel ist sie als flexible Kanüle 5 ausgebildet. Die Einführeinrichtung 5 wird von einer Einstecheinrichtung 15 durchragt, die als dünne Nadel 15 ausgebildet ist, deren Biegesteifigkeit ausreicht, um die Einstecheinrichtung 15 gemeinsam mit der sie umgebenden, sich anschmiegenden Einführeinrichtung 5 durch die Hautoberfläche in subkutanes Gewebe einzustechen und dadurch die Einführeinrichtung 5 einzuführen. In bevorzugten Ausführungen ist an der Unterseite U der Basis 1, 2 ein Klebepad für eine Fixierung des Insertionskopfs auf dem Gewebe, vorzugsweise der Hautoberfläche, angebracht.

Für die Schwenkbeweglichkeit der Einführeinrichtung 5 gemeinsam mit der Einstecheinrichtung 15 sorgt ein Halteelement 17a, das auf zwei gegenüberliegenden Seiten je einen Wellenstumpf 40 eines Drehgelenks bildet. Die Achsen der Wellenstümpfe 40 fallen zusammen. Die erste Griffkomponente 10 bildet die Lagerstellen für die Wellenstümpfe 2 in Form von Buchsen oder gegebenenfalls auch offener Lageraugen.

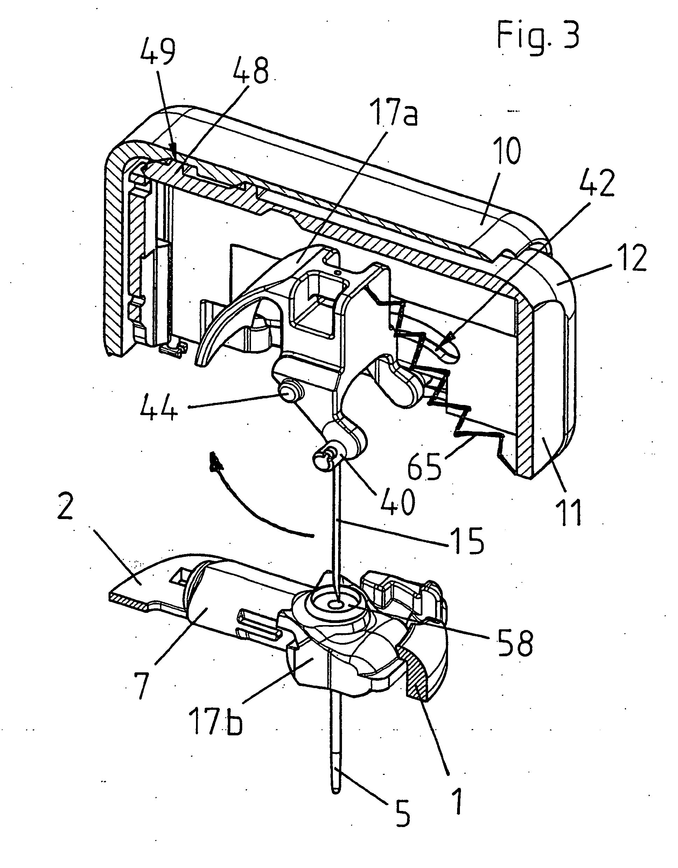

Die Einstecheinrichtung 15 ist fest mit der Halteelement 17a verbunden, während die Kanüle 5 fest mit einem Kanülengehäuse 17b verbunden ist, welches eine Zuführung 7 für eine Medikamentenflüssigkeit, beispielsweise Insulin, aufweist. Die Zuführung 7 ragt in etwa rechtwinklig zur Längsachse der Einführeinrichtung 5 von dem Kanülengehäuse 17b ab. In Verlängerung der Längsachse der Kanüle 5 weist das Kanülengehäuse 17b an seinem der Kanüle 5 abgewandten Ende ein Septum 58 (in Fig. 3 erkennbar) auf, welches von der Einstechnadel 15 durchdrungen ist. Auf diese Weise sind die Kanüle 5 und das Kanülengehäuse 17b kraftschlüssig mit dem Halteelement 17a bzw. der daran gehaltenen Einstechnadel 15 verbunden und können durch Drehung des Halteelements 17a um die Drehachsen der Wellenstümpfe 40 als Einheit 5, 6, 7, 15, 17b geschwenkt werden.

Wie weiter zu erkennen ist, ist die bewegliche zweite Griffkomponente 12 an jeder ihrer beiden Längsseiten mit einer Kulissenführung 42 versehen, in welche jeweils ein zapfenförmiger Kulissenstein 44 des Halteelements 17a eingreift. Von den beiden Kulissenführungen 42 ist aufgrund der geschnittenen Darstellung nur eine gezeigt, und von den Kulissensteinen 44 ist nur einer erkennbar, da der andere in der Darstellung durch das Halteelement 17a verdeckt ist.

Im Falle eines Einschiebens der gegenüber der Basis 1, 2 beweglichen zweiten Griffkomponente 12 in Richtung des Richtungspfeils in die gegenüber der Basis 1, 2 feststehende erste Griffkomponente 10, wobei die erste Griffkomponente 10 bei dieser Bewegung die zweite Griffkomponente 12 führt, werden die Kulissenführungen 42 entlang der Gleitsteine 44 verschoben, so dass die Verschiebebewegung der zweiten Griffkomponente 12 in eine Drehbewegung des Halteelements 17a um die Achsen der Wellenstümpfe 40 und damit in eine Schwenkbewegung der Einführeinrichtung 5 und der Einstecheinrichtung 15 sowie der Zuführung 7 um diese Achsen übertragen wird.

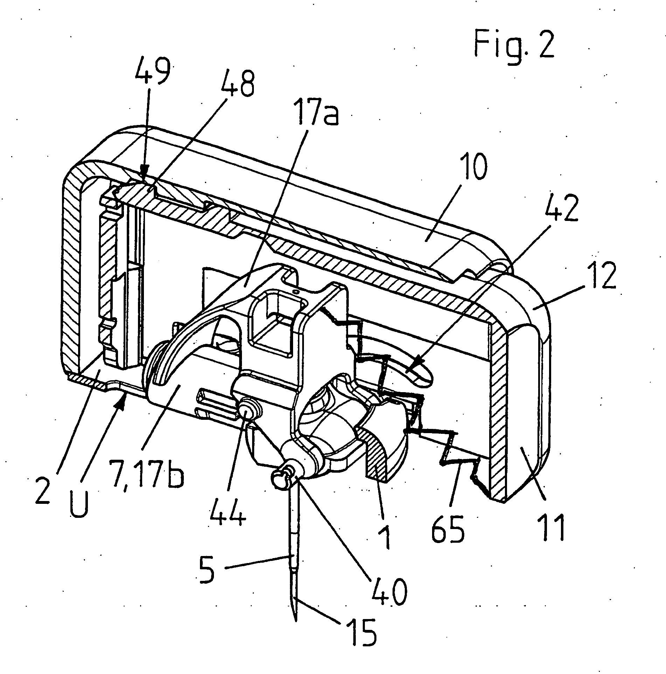

Dabei findet die Schwenkbewegung, welche die Einführeinrichtung 5 und die Einstecheinrichtung 15 aus deren in Figur 1 gezeigten Schutzposition in die in der Fig. 2 gezeigte Einführposition überführt, entgegen der Federkraft einer Zugfeder 65, welche zwischen dem Halteelement 17a und der zweiten Griffkomponente 12 angeordnet ist und mit zunehmender Schwenkbewegung zunehmend vorgespannt wird, statt.

In der Schutzposition weisen die Einführeinrichtung 5 und die Einstecheinrichtung 15 gegenüber der Unterseite U der Basis 1, 2 einen Winkel von etwa 10° auf. Die Einführeinrichtung 5 und der in die gleiche Richtung über das Halteelement 17a hinaus stehende Abschnitt der Einstecheinrichtung 15 sind in der gemeinsamen Schutzposition in einem vom ersten Griffteil 10 und der Basis 1, 2 im Wesentlichen umschlossenen Aufnahmeraum 14 aufgenommen. Mit in der Schutzposition befindlicher Einführ- und Einstecheinrichtung 5, 15 ist gewährleistet, dass der Benutzer sich nicht an der Einstecheinrichtung 15 verletzen und umgekehrt die Einführeinrichtung 5 und die Einstecheinrichtung 15 nicht durch unachtsame Handhabung beschädigt oder kontaminiert werden können. Da die Basis 1, 2 lediglich einen schmalen Durchtrittschlitz für die Einführ- und Einsticheinrichtung 5, 15 aufweist, bildet die Aufnahme 14 hier auch einen Sichtschutz, so dass der Benutzer die Einstecheinrichtung 15 von der Oberseite des Insertionskopfs und auch bei seitlichem Blickwinkel nicht erkennen kann. Ein bevorzugterweise an der Unterseite der Basis 1, 2 vorhandenes Klebepad ist ebenfalls mit einem Durchtrittsschlitz für die Einführeinrichtung 5 und die Einstecheinrichtung 15 versehen.

Um die Einführeinrichtung 5 in das Körpergewebe bis unter oder gegebenenfalls nur in die Haut einzuführen, greift der Benutzer den Insertionskopf am Griff 10, 12 zwischen Daumen und Zeigefinger. Die Griffkomponenten 10 und 12 sind je mit einer geeignet geformten, seitlichen Grifffläche 11 versehen. Durch Zusammendrücken der Griffkomponenten 10 und 12 werden, wie bereits beschrieben wurde, die Kulissenführungen 42 in den Seitenwände 44 des Halteelements 17a verschoben, so dass die Einführeinrichtung 5 und die Einstecheinrichtung 15 in die in Fig. 2 dargestellte Einführposition geschwenkt werden. Bei Erreichen dieser Position verrasten die beiden Griffkomponenten 10, 12 irreversibel miteinander, indem eine am zweiten Griffteil 12 gebildete Rastnase 48 federnd in einen an der ersten Griffkomponenten gebildeten entsprechenden Hinterschnitt 49 eingreift, so dass sie nicht wieder auseinander bewegt werden können. Gleichzeitig wird die ersten Griffkomponente 10, welche in der in Fig. 1 gezeigten Situation mit der Basis 1, 2 unbeweglich, aber lösbar verbunden ist, durch Einschieben der zweiten Griffkomponente 12 in die erste Griffkomponente 10 von der Basis gelöst, indem an der ersten Griffkomponente 10 gebildete federnde Rastnasen (nicht gezeigt) ausser Eingriff mit entprechenden Gegenelementen an der Basis 1, 2 gebracht werden. In diesem Zustand sind der Griff 10, 12, das Halteelement 17a und die Einstecheinrichtung 15 nur noch über die Reibung zwischen der Einstecheinrichtung 15 und dem Septum 58 des Kanülengehäuses 17b mit der Basis 1, 2, der Einführeinrichtung 5 und dem Kanülengehäuse 17b verbunden.

Ebenfalls verrastet bei Erreichen der Einführposition das Kanülengehäuse 17b mittels Rastmitteln (nicht dargestellt) irreversibel mit der Basis 1, 2, so dass beide Bauteile 1, 2, 17b in der in der Einführposition zueinander eingenommenen Position fest und irreversibel miteinander verbunden werden. Wie solche Rastmittel ausgebildet sein können, z.B. in Form von federnden Rastnasen, welche in entsprechenden Hinterschnitte einrasten, ist dem Fachmann bekannt und muss deshalb hier nicht im Einzelnen erläutert werden.

Der Abstand der Gleitsteine 44 von der Schwenkachse und die Bahnkurven der Kulissenführungen 42 sind im vorliegenden Fall derartig gewählt, dass eine Verschiebung der zweiten Griffkomponente 12 um wenige Millimeter, beispielsweise 4 oder 5 mm, eine Schwenkbewegung der Einführeinrichtung 5 und der Einstecheinrichtung 15 von der Schutzposition in die Einführposition um einen Schwenkwinkel von etwa 80° in bewirkt, wobei die Einführeinrichtung 5 und die Einstecheinrichtung 15 in der Einführposition über die Unterseite U der Basis 1, 2 in etwa rechtwinklig vorragen.

Für die Platzierung des Insertionskopfs auf einer Gewebeoberfläche und die Einführung der Einführeinrichtung 5 in das Gewebe hält der Benutzer den Insertionskopf am Griff 10, 12 und bewegt ihn auf die Gewebeoberfläche zu. Dabei durchsticht die Einstecheinrichtung 15 die Gewebeoberfläche, vorzugsweise die menschliche Haut, und dringt in die Haut ein. Die sich anschmiegende Einführeinrichtung 5 dringt gemeinsam mit der Einstecheinrichtung 15 ein, bis der Insertionskopf mit seiner Unterseite U, d.h. mit der Unterseite U der Basis 1, 2, auf der Gewebeoberfläche aufsetzt und dabei z.B. adhäsiv auf der Hautoberfläche fixiert wird, vorzugsweise mittels einer Klebeschicht oder eines Klebepads.

Für die Verabreichung des Medikaments muss nach der Platzierung des Insertionskopfes auf dem Gewebe der Griff 10, 12 mit dem Haltelement 17a und der Einstecheinrichtung 15 entfernt und die Zuführung 7 über einen mit der Zuführung 7 zusammenwirkenden Konnektor mit einem Medikamentenreservoir, vorzugsweise einer Medikamentenpumpe, verbunden werden.

Um dies zu bewerkstelligen, wird der Griff 10, 12 mit einer Hand ergriffen und entgegen der Richtung des Einführens der Einführeinrichtung 5 in das Gewebe von der Basis 1, 2 wegbewegt, wodurch die Einstecheinrichtung 15 aus der Einführeinrichtung 5 und dem Kanülengehäuse 17b herausgezogen wird und den Strömungsquerschnitt der Einführeinrichtung 5 freigibt. Die Eintrittsöffnung der Einstecheinrichtung 15 in das Kanülengehäuse 17b, welche von einem Septum 58 gebildet wurde, wird nach dem Herausziehen der Einstecheinrichtung 15 durch das Septum 58 verschlossen. Beim Herausziehen der Einstecheinrichtung 15 aus der Einführeinrichtung 5 wird der Strömungsquerschnitt der Einführeinrichtung 5 automatisch fluidisch mit der Zuführung 7 verbunden. Diesbezüglich kann der Insertionskopf wie beispielsweise in der DE 198 21 723 C1 und der DE 10 2004 039 408.3 beschrieben ausgebildet sein.

Fig. 3 zeigt die beiden voneinander gelösten Baugruppen des Insertionskopfs, nämlich die Basis 1, 2 mit dem Kanülengehäuse 17b und der Einführeinrichtung 5 einerseits und den Griff 10, 12 mit dem Halteelement 17a und der Einstecheinrichtung 15 andererseits, in zueinander ausgerichteter Position, in der die Längsachse der Einführeinrichtung 5 und die Längsachse der Einstecheinrichtung 15 miteinander fluchten.

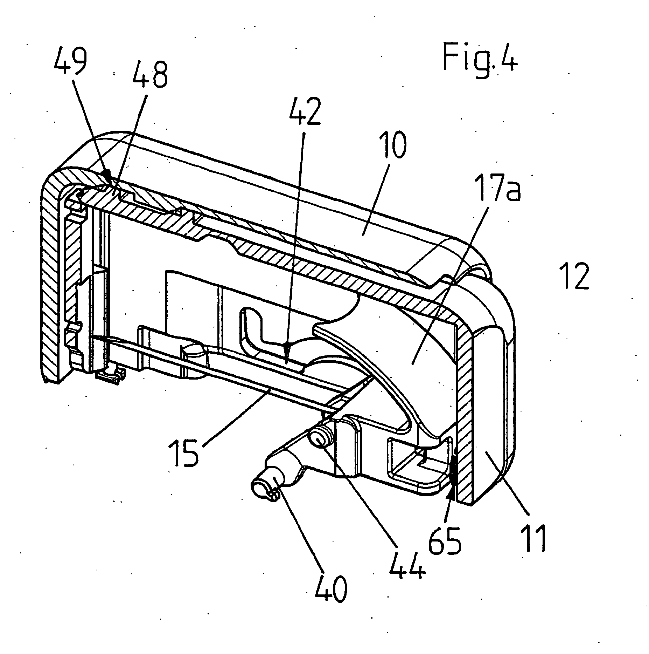

Direkt nachdem die Einstecheinrichtung 15 das Septum 58 verlassen hat, wird sie automatisch zusammen mit dem in der zweiten Griffkomponente 12 gelagerten Halteelement 17a durch die vorgespannte Zugfeder 65 in der in Fig. 3 durch einen Drehrichtungspfeil angegebenen Richtung in die Schutzposition zurückgeschwenkt, so dass sie wieder innerhalb der Begrenzungen des durch die beiden Griffkomponenten 10, 12 gebildeten Gehäuses angeordnet ist. Diese Situation ist in Fig. 4 dargestellt.

Die in Fig. 3 einzeln dargestellte Basis 2 und das Kanülengehäuse 17b mit der daran befestigten Kanüle 5 verbleiben auf der Gewebeoberfläche und bilden in diesem Sinne ein Verbleibteil. Der Griff 10, 12 mit der darin in der Schutzposition angeordneten und durch das Halteelement 17a gehaltenen Einstecheinrichtung 15 hingegen wird entsorgt. Das Verbleibteil kann somit vorteilhaft flach sein und stört nicht, wenn es unter der Kleidung getragen wird. Die Flexibilität der Einführeinrichtung 5 ist derart, dass die Einführeinrichtung 5 im eingeführten Zustand als nicht störend empfunden wird, aber doch ausreichend stabil ist, um eine Versorgung mit dem Medikament sicher zu gewährleisten.

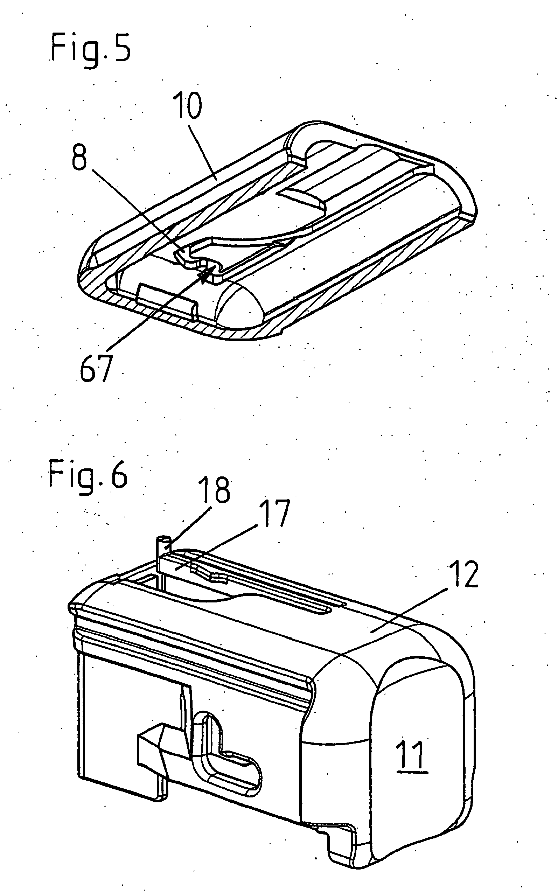

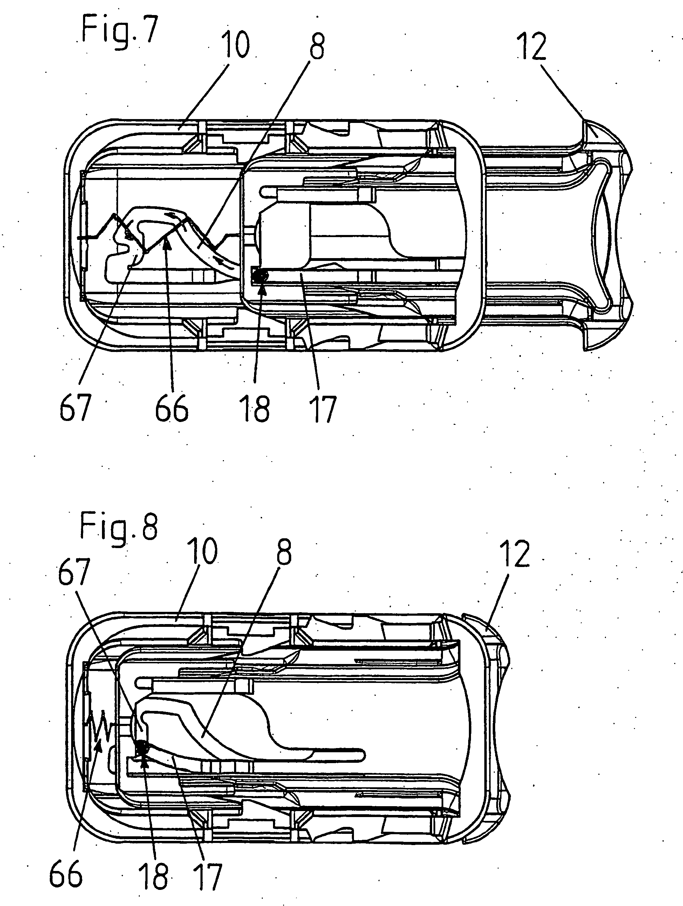

Fig. 5 zeigt den oberen Abschnitt eines von zwei ineinanderschiebbaren Gehäuseteilen 10, 12 eines zweiten erfindungsgemässen Insertionskopfes im perspektivischen Horizontalschnitt schräg von unten. Es handelt sich hierbei um den oberen Abschnitt des ersten, äusseren Gehäuseteils 10, in welches das zweite, innere Gehäuseteil 12, welches in einer perspektivischen Seitenansicht in Fig. 6 dargestellt ist, bei vollständig montiertem Insertionskopf zumindest teilweise eingeschoben ist. Abgesehen von den im Folgenden erläuterten Unterschieden weist der zweite erfindungsgemässe Insertionskopf praktisch den gleichen Aufbau und die gleichen Funktionalitäten wie der in den Figuren 1 bis 4 beschriebene Insertionskopf auf. Der wesentlichste Unterschied zum zuvor beschriebenen Insertionskopf besteht darin, dass bei der hier vorliegenden Ausführungsform die beiden Gehäuseteile 10, 12 nach dem Zusammenschieben und dem dadurch bewirkten Ausschwenken der Einführeinrichtung und der Einstecheinrichtung von der Schutzposition in die Einführposition mittels eines so genannten "Kugelschreibermechanimus" vorübergehend in der die Einführposition repräsentierenden zusammengeschobenen Position miteinander verrastet werden, und nach dem Applizieren der Einführeinrichtung und dem anschliessenden Trennen der Gehäuseteile 10, 12 mit der Einstechvorrichtung 15 von der Einführeinrichtung 5, der Basis 1, 2 und dem Kanülengehäuse 17b, welche an der Applikationsstelle verbleiben, durch ein erneutes Zusammendrücken der Gehäuseteile 10, 12 in Zusammenschieberichtung und anschliessende Entlastung derselben wieder durch die Kraft einer beim Zusammenschieben zunehmend vorgespannten Druckfeder 66 (siehe Figuren 7 bis 9) auseinandergeschoben werden, wobei die Einstecheinrichtung dann automatisch wieder in eine Schutzposition innerhalb der Begrenzungen des von den beiden Gehäuseteilen 10, 12 gebildeten Gehäuses eingeschwenkt wird.

Zur Realisierung dieses "Kugelschreibermechanismus" weist der erste Gehäuseteil 10 auf der Unterseite seiner oberen Begrenzungswandung eine Führungsnut 8 auf, in welche ein auf der Oberseite des zweiten Gehäuseteils 12 von einem federelastischen Arm 17 getragener Führungszylinder 18 eingreift und beim Zusammenschieben der beiden Gehäuseteile 10, 12 geführt wird. Wie aus einer Zusammenschau der Figuren 7 und 8, welche eine Ansicht von unten auf die beiden Gehäuseteile 10, 12 ohne Einführeinrichtung, Einstecheinrichtung, Basis und Kanülengehäuse einmal in einer Grundposition zueinander, welche der Schutzposition von Einführ- und Einstecheinrichtung entspricht (Fig. 7), und einmal in einer im Wesentlichen vollständig zusammengeschobenen Position zueinander, welche der Einführposition von Einführ- und Einstecheinrichtung entspricht (Fig. 8), zeigen, wird der Führungszylinder 18, dessen Position in den Figuren 7 bis 9 der besseren Nachvollziehbarkeit in Form eines schwarzen Punktes dargestellt ist, beim Zusammenschieben der beiden Gehäuseteile 10, 12 entgegen der Kraft der Druckfeder 66 unter horizontaler Auslenkung entgegen der Federkraft des federelastischen Arms 17 in eine hintere Nische 67 der Führungsnut 8 verschoben (siehe Pfeile in Fig. 7), in welcher er nach dem Entlasten der beiden Gehäuseteile 10, 12 durch die Kraft der Druckfeder 66 arretiert wird (Fig. 8). Hierdurch wird bei vollständig montiertem Insertionskopf auch die Einführ- und Einstechvorrichtung, welche beim Zusammenschieben über die mechanische Kopplung von der Schutzposition in die Einführposition geschwenkt wird, in der Einführposition arretiert und der Insertionskopf applikationsbereit gemacht.

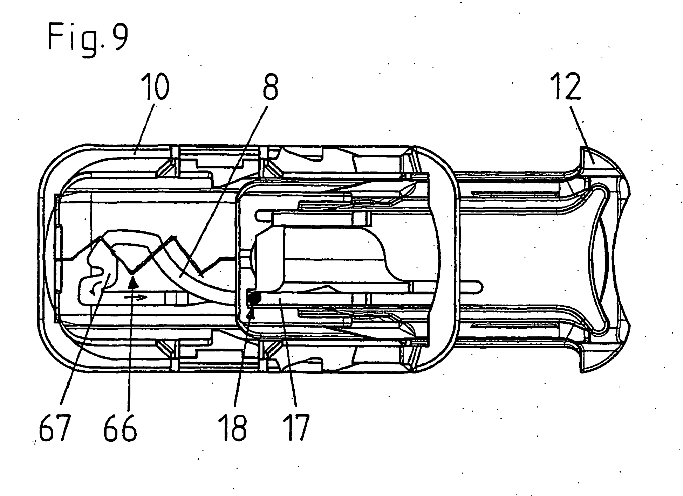

Nach dem Applizieren des Insertionskopfs durch Einstechen der Einführeinrichtung und der Einstecheinrichtung in ein Gewebe und dem Entfernen der beiden Gehäuseteile 10, 12 mit der daran befestigten Einstecheinrichtung von den an der Applikationsstelle verbleibenden Komponenten des Insertionskopfes wird die Arretierung des Führungszylinders 18 in der Nische 67 durch erneutes Zusammendrücken der beiden Gehäuseteile 10, 12 in Zusammenschieberichtung gelöst, da hierbei infolge der Federkraft des federelastischen Arms 18 der Führungszylinder 18 in einen anderen Abschnitt der Führungsnut 8 überführt wird, entlang welchem er unter einem durch die Kraft der Druckfeder 66 bewirkten Auseinanderschieben der beiden Gehäuseteile 10, 12 in die Grundposition und unter gleichzeitigem Zurückschwenken der Einstecheinrichtung in die Schutzposition in seine Ausgangsposition zurückgeführt wird (siehe Pfeile in Fig. 9). In diesem Zustand kann das Gehäuse 10, 12 mit der darin gegen Zugriff geschützten kontaminierten Einstecheinrichtung problemlos und ohne Verletzungsgefahr entsorgt werden.

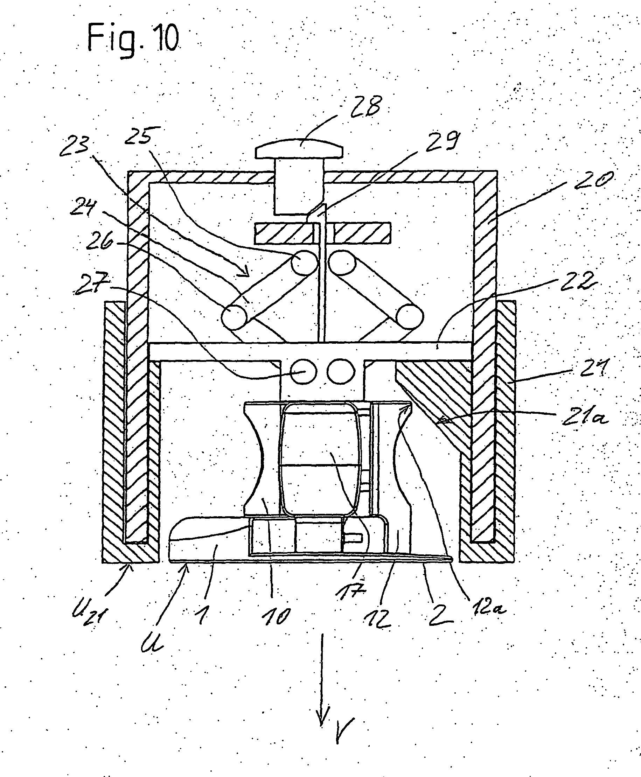

Fig. 10 zeigt ein System bestehend aus einem erfindungsgemässen Insertionskopf ähnlich den in den Figuren 1 bis 9 gezeigten und einer speziell darauf angepassten Insertionsvorrichtung, im Folgenden als Inserter bezeichnet, welche der Platzierung des Insertionskopfs auf dem Gewebe dient, so dass der Benutzer den Insertionskopf bei der Platzierung nicht zwischen den Fingern greifen muss. Insbesondere hält der Benutzer bei der Überführung der Einführ- und Einstecheinrichtung 5, 15 in die Einführposition den Insertionskopf nicht am Griff. Diese Aktivierung des Insertionskopfs wird mit Hilfe des Inserters vorgenommen. Der Benutzer wird daher noch besser vor Stichverletzungen durch die Einstecheinrichtung 15 geschützt und die Einführeinrichtung 5 und die Einstecheinrichtung 15 werden noch besser vor Beschädigungen und Kontamination durch unachtsame Handhabung geschützt, indem selbst bei in die Einführposition ausgeklappter Einführ- und Einstecheinrichtung ein unbeabsichtigtes Inkontaktkommen mit diesen durch den Inserter verhindert wird.

Der Inserter weist ein Insertergehäuse 20 auf, das als Hülsenteil mit einem Boden gebildet ist und von außen betrachtet im Wesentlichen die Form eines Topfs aufweist. Das Insertergehäuse 20 nimmt eine Halteeinrichtung und einen Antrieb für den Insertionskopf auf. Die Halteeinrichtung umfasst eine Haltefeder, beispielsweise eine Blattfeder, die den Insertionskopf in der in Fig. 10 gezeigten Ausgangsposition relativ zu dem Insertergehäuse 20 hält. In dem Halteeingriff hintergreift die Haltefeder eine an dem Griff 10, 12 geformte Haltestruktur 17. Der Halteeingriff ist gegen die rückstellende Elastizitätskraft der Haltefeder lösbar.

Der Antrieb umfasst ein Vortriebselement 22, das in und gegen eine Vortriebsrichtung V linear beweglich in dem Insertergehäuse 20 angeordnet ist. Die Vortriebsrichtung V fällt mit einer zentralen Längsachse des Insertergehäuses 20 zusammen. Der Antrieb umfasst ferner einen Krafterzeuger 23, der in die Vortriebsrichtung V auf das Vortriebselement 22 wirkt. Der Krafterzeuger 23 umfasst zwei Paare von gelenkig miteinander verbundenen Schenkeln 24, wobei die beiden Paare von Schenkeln 24 symmetrisch bezüglich der zentralen Längsachse, d.h. symmetrisch zur Vortriebsrichtung V des Insertergehäuses 20 angeordnet sind. Jedes der Schenkelpaare ist in einem zum Insertergehäuse 20 ortsfesten Drehgelenk 25 aufgehängt. Die beiden Schenkel 24 des jeweiligen Schenkelpaars sind in einem freien Drehgelenk 26 miteinander drehbeweglich verbunden. Ferner ist der von dem ortsfesten Gelenk 25 abgewandte Schenkel 24 jeweils in einem Drehgelenk 27 mit dem Vortriebselement 22 verbunden. Nicht dargestellte Federn oder gegebenenfalls auch nur eine Feder spannt diese Schenkel-Gelenk-Vortriebselement-Anordnung in die Vortriebsrichtung V. Die Anordnung aus Schenkeln 24 und Gelenken 25, 26 und 27 führt das Vortriebselement 22; zusätzlich oder stattdessen könnte die Mantelinnenfläche des Insertionsgehäuses 23 das Vortriebselement 22 führen. Ferner ist ein Blockierglied 29 vorgesehen, das mit dem Insertergehäuse 20 in einem Blockiereingriff ist, der eine Vortriebsbewegung des Vortriebselements 22 verhindert. Das Blockierglied 29 kann den Blockiereingriff mit der von dem Insertergehäuse 20 gebildeten Hüllstruktur oder ebenso mit einer damit in Bezug auf die Vortriebsrichtung V fest verbundenen sonstigen Struktur bilden. Der Blockiereingriff ist durch Betätigung eines druckknopfartigen Auslösers 28 lösbar.

Der Inserter umfasst ferner ein Aktivierungsglied 21, das mit dem Insertergehäuse 20 in und gegen die Vortriebsrichtung V beweglich verbunden ist. Das Aktivierungsglied 21 bildet in Bezug auf das Insertergehäuse 20 eine Muffe, so dass insgesamt ein zweiteiliges, teleskopierbares Insertergehäuse mit Gehäuseteilen 20 und 21 erhalten wird. Der Unterscheidung in funktionaler Hinsicht wegen wird das Gehäuseteil 21 jedoch weiterhin als Aktivierungsglied bezeichnet. Das Aktivierungsglied 21 bildet die Unterseite U21 des Inserters, mit welcher der Inserter für die Platzierung des Insertionskopfs auf der Gewebeoberfläche aufsetzbar ist und vorzugsweise auch aufgesetzt wird. In der in Fig. 10 vom Insertionskopf eingenommenen Ausgangsposition weisen die Unterseite U21 des Inserters und die Unterseite U des gehaltenen Insertionskopfs jeweils in die Vortriebsrichtung V, die für die beiden Unterseiten zumindest im Wesentlichen eine Flächennormale bildet.

Das Aktivierungsglied 21 umfasst ein äußeres Hülsenteil und ein inneres Hülsenteil, die an der Unterseite U21 miteinander verbunden sind und zwischen sich einen Ringspalt freilassen. Das Insertergehäuse 20 ragt in diesen Ringspalt und führt das Aktivierungsglied 21 gleitbeweglich.

In dem in Fig. 10 gezeigten Zustand nimmt das Aktivierungsglied 21 relativ zu dem Insertergehäuse 20 eine eingefahrene Position ein und der Inserter weist eine in Vortriebsrichtung V gemessen kürzeste Länge auf. In diesem Zustand des Inserters wird der Insertionskopf eingesetzt, d.h. in den Halteeingriff mit der Halteeinrichtung des Inserters gebracht. Anstatt den Insertionskopf einzusetzen, kann der Inserter auch über den auf einer Auflage liegenden Insertionskopf gestülpt werden. Die Position und Geometrie der Halteeinrichtung ist so gewählt, dass der Halteeingriff bei dem Aufstülpen automatisch entsteht. Unmittelbar nach dem Aufnehmen des Insertionskopfs, beispielsweise durch Einsetzen, befindet sich die Einführeinrichtung 5 des Insertionskopfs in ihrer Schutzposition. In diesem Sinne ist der Insertionskopf noch inaktiv. Der Inserter ist mit Mitteln ausgestattet, nämlich dem Aktivierungsglied 21, durch deren Betätigung die Einführeinrichtung in die Einführposition bewegt und auf diese Weise der Insertionskopf aktiviert werden kann.

Für die Aktivierung bilden das Aktivierungsglied 21 und der Insertionskopf miteinander ein Gelenk, im Ausführungsbeispiel ein Kurvengelenk. Die beiden Gelenkelemente des Gelenks sind eine Führungskurve 21a, die das Aktivierungsglied 21 bildet, und ein von der beweglichen Griffkomponente 12 gebildetes Eingriffselement 12a. In der Kopplung, über die das Aktivierungsglied 21 auf die Einführeinrichtung 5 einwirkt, bildet die bewegliche Griffkomponente 12 ein Eingangs- bzw. Empfangsglied des Insertionskopfs. Wird das Aktivierungsglied 21 relativ zu dem Insertergehäuse 20 in die Vortriebsrichtung V bewegt, gleitet die Führungskurve 21a über das Eingriffselement 12a, d. h. über die das Eingriffselement 12a bildende Kontaktfläche des Empfangsglieds, d. h. der beweglichen Griffkomponente 12. Durch den Druckkontakt und den zur Vortriebsrichtung V geneigten Verlauf der Führungskurve 21a bewegt sich die Griffkomponente 12 quer zu der Vortriebsrichtung V auf die andere Griffkomponente 10 zu, und die Einführeinrichtung 5 schwenkt wie zum Insertionskopf als solchem beschrieben in die Einführposition. Die bewegliche Griffkomponente 12 bildet das Eingriffselement 12a an ihrem von der Basis 2 abgewandten oberen Ende, im Ausführungsbeispiel mit ihrer äußeren Kante. Die Führungskurve 21a ist der Unterseite U21 des Inserters zugewandt. Die Neigung ist so gewählt, dass sich die Führungskurve 21a von einem von der Unterseite U21 abgewandten Ende in Vortriebsrichtung V von dem Insertionskopf bzw. der im aktivierten Zustand ausgeschwenkten Einführeinrichtung 5 oder der zentralen Längsachse des Inserters weg neigt. Der Neigungswinkel ist überall konstant, die Führungskurve 21a ist eine Schräge, d. h. eine schräge Linie oder Fläche.

Für die praktische Handhabung bietet es sich an, dass der Benutzer den Inserter nach dem Aufnehmen des Insertionskopfs mit der einen Hand am Aktivierungsglied 21 hält, beispielsweise indem er das Aktivierungsglied 21 umgreift, und mit der anderen Hand das Insertergehäuse 20 relativ zu dem gehaltenen Aktivierungsglied 21 gegen die Vortriebsrichtung V zieht. Auch dies wird als Betätigung des Aktivierungsglieds verstanden. Das Vortriebselement 22 und der Krafterzeuger 23 bewegen sich gemeinsam mit dem Insertergehäuse 20 relativ zu dem Aktivierungsglied 21. Der von der Halteeinrichtung in der Ausgangsposition gehaltene Insertionskopf wird mitgenommen, d. h. bewegt sich gleichermaßen relativ zu dem Aktivierungsglied 21 entgegen der Vortriebsrichtung V. Das Eingriffselement 12a gleitet längs der Führungskurve 21a. Über diese auf reinem Druckkontakt beruhende Schnittstelle wird die bewegliche Griffkomponente 12 quer zu der Vortriebsrichtung V bewegt, und die Einführeinrichtung 5 schwenkt in die Einführposition. Der Insertionskopf ist am Ende der Ausfahrbewegung, die das Insertionsgehäuse 20 und das Aktivierungsglied 21 relativ zueinander ausführen, aktiviert.

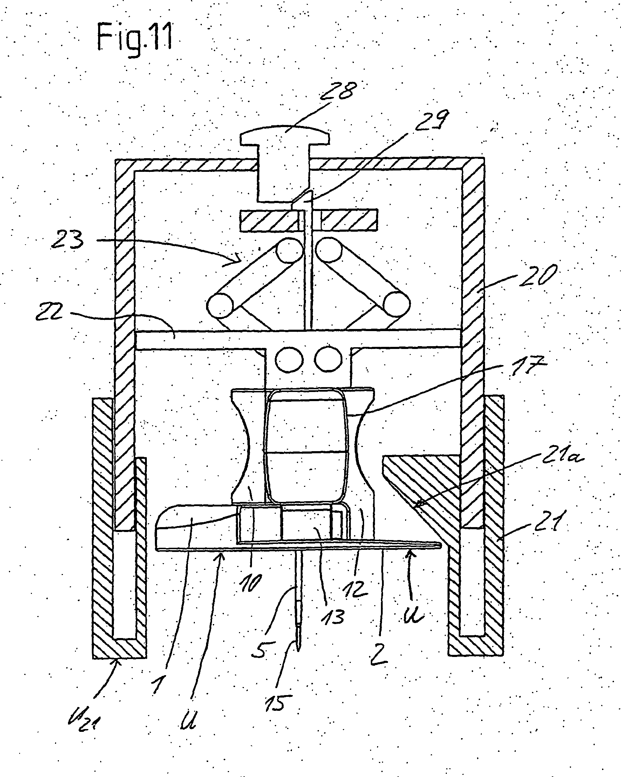

Fig. 11 zeigt das System aus Inserter und Insertionskopf in dessen aktiviertem Zustand. Das Insertergehäuse 20 und das Aktivierungsglied 21 nehmen relativ zueinander die ausgefahrene Position ein. In dem ausgefahrenen Zustand umgeben die Wände des Insertergehäuses 20 und des Aktivierungsglieds 21 den aktivierten Insertionskopf bis über das freie Ende der Einführeinrichtung 5 und der Einstecheinrichtung 15 hinaus, d. h. die Spitze der Einstecheinrichtung 15 steht ein kleines Stück weit hinter der Unterseite U21 des Inserters zurück.

Das Insertergehäuse 20 und das Aktivierungsglied 21 sind in der ausgefahrenen Position relativ zueinander blockiert. Relativbewegungen in oder gegen die Vortriebsrichtung V sind in dem blockierten Zustand nicht möglich. Bei Erreichen der ausgefahrenen Position blockieren sich das Insertergehäuse 20 und das Aktivierungsglied 21 aneinander automatisch.

Für die Platzierung des Insertionskopfs setzt der Benutzer den Inserter auf der Hautoberfläche auf. Bei aufgesetztem Inserter drückt der Benutzer auf den Auslöser 28. Der Auslöser 28 wirkt über ein Kurvengelenk, im Ausführungsbeispiel ein einfaches Paar von Schrägen, auf das Blockierglied 29. Unter der Einwirkung des Auslösers 28 bewegt sich das Blockierglied 29 aus dem Blockiereingriff mit dem Insertergehäuse 20, so dass sich das Vortriebselement 22 unter der Einwirkung des Krafterzeugers 23 in die Vortriebsrichtung V bewegen kann. Der Krafterzeuger 23 beschleunigt das Vortriebselement 22 schlagartig. Das Vortriebselement 22 wirkt auf den Insertionskopf wie ein Hammer. Im ersten Abschnitt der Vortriebsbewegung federt die Haltefeder aus dem Halteeingriff mit der Haltestruktur 17 des Insertionskopfs, d.h. der Halteeingriff löst sich. Die Beschleunigung des Vortriebselements 22 in die Vortriebsrichtung V ist so groß, dass der reine Druckkontakt zwischen dem Vortriebselement 22 und dem Insertionskopf sicher erhalten bleibt, bis die Unterseite U des Insertionskopfs auf der gleichen Höhe wie die Unterseite U21 des Inserters und somit auf der Gewebeoberfläche platziert ist. Bereits zuvor durchdringt die Einstecheinrichtung 15 die Hautoberfläche, dringt in das Gewebe ein und nimmt dabei die Einführeinrichtung 5 mit.

Nachdem der Insertionskopf auf der Hautoberfläche platziert und der Inserter entfernt ist, greift der Benutzer den Griff 10, 12 und zieht ihn entgegen der Einführungsrichtung von der Basis 1, 2 ab. Dabei wird die Einstecheinrichtung 15 aus der Einführeinrichtung 5 heraus- und von der Basis 1, 2 abgezogen und klappt sodann durch eine Feder betätigt automatisch in eine Schutzposition in den Griff 10, 12 ein, gegebenenfalls nach einem vorherigen erneuten Zusammendrücken der beiden Griffkomponenten 10, 12 in Zusammenschieberichtung und anschliessendes Entlasten derselben.

In einer vorteilhaften Modifikation des Inserters bleibt, um auch das Herausziehen der Einstecheinrichtung 15 zu automatisieren, der Halteeingriff zwischen der Halteeinrichtung des Inserters und der Haltestruktur 17 des Insertionskopfs bestehen und wird nicht, wie im zuvor beschriebenen Ausführungsbeispiel, durch die Beschleunigung des Vortriebselements 22 gelöst. In einer derartigen Modifizierung kann die Halteeinrichtung insbesondere ortsfest mit dem Vortriebselement 22 verbunden sein, so dass sie dessen Ausstoßbewegung in die Vortriebsrichtung V mitmacht. Um den Halteeingriff zu lösen, kann der Inserter mit einem Abstreifer ausgestattet sein, der nach dem Wegnehmen des Inserters vom Gewebe bei einem Zusammenschieben von Insertergehäuse 20 und Aktivierungsglied 21 automatisch den Insertionskopf aus dem Halteeingriff löst, gegebenenfalls nach einem vorherigen automatischen oder durch eine zusätzliche Manipulation bewirkten erneuten Zusammendrücken in Zusammenschieberichtung mit anschliessender Entlastung der beiden Griffkomponenten 10, 12. Alternativ kann solch ein Abstreifer auch vollkommen unabhängig vom Aktivierungsglied 21 vorgesehen und separat betätigbar sein, um den Halteeingriff zu lösen.

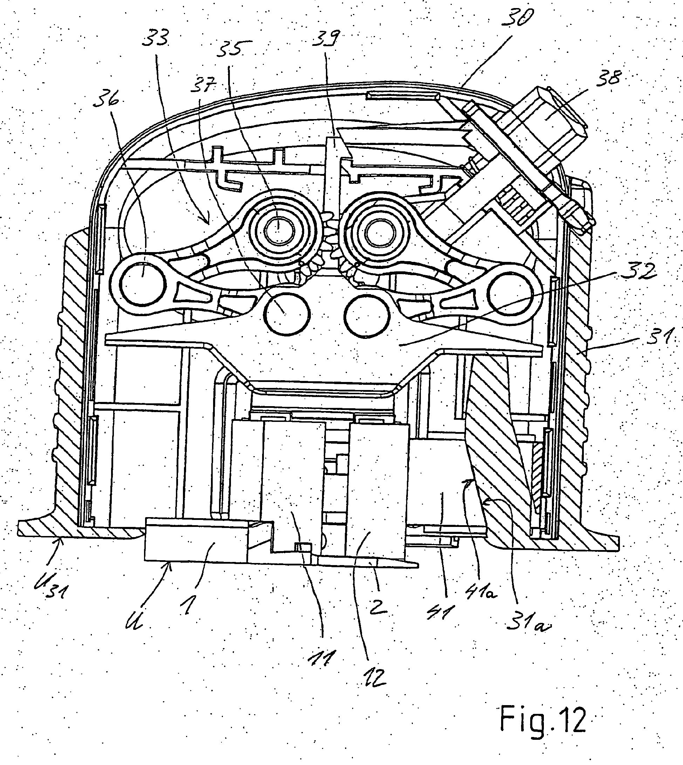

Die Figuren 12 bis 14 zeigen ein zweites Ausführungsbeispiel eines aus einem erfindungsgemässen Insertionskopf und einem Inserter bestehenden Systems. Der Insertionskopf kann der gleiche wie im ersten Ausführungsbeispiel sein. Nur bei dem Inserter handelt es sich um eine Modifikation. Diejenigen Komponenten des Inserters des zweiten Ausführungsbeispiels, die in Bezug auf ihre Funktion mit den Komponenten des Inserters des ersten Ausführungsbeispiels vergleichbar sind, werden jeweils mit den um die Zahl zehn erhöhten Bezugszeichen des ersten Ausführungsbeispiels belegt. So gelten insbesondere zum Insertergehäuse 30 und dem Aktivierungsglied 31, was deren Form und Verbindung sowie Relativbeweglichkeit anbetrifft, die Ausführungen zum ersten Ausführungsbeispiel. Grundsätzlich das Gleiche gilt auch in Bezug auf das Vortriebselement 32, die Halteeinrichtung und den Krafterzeuger 33 sowie den Auslöser 38 und das Blockierglied 39. Soweit im Folgenden auf Unterschiede nicht hingewiesen wird und sich auch aus den Figuren nichts anderes ergibt, gelten die Ausführungen zum ersten Ausführungsbeispiel gleichermaßen auch für das zweite Ausführungsbeispiel.

Der Inserter des zweiten Ausführungsbeispiels unterscheidet sich von dem Inserter des ersten Ausführungsbeispiels im Wesentlichen im Hinblick auf das Gelenk, über welches das Aktivierungsglied 31 auf den Insertionskopf wirkt, um diesen durch die Aufziehbewegung des Insertergehäuses 30 relativ zu dem Aktivierungsglied 31 zu aktivieren. Im zweiten Ausführungsbeispiel bildet der Inserter selbst das Gelenk, nämlich mit zwei Gelenkelementen 31a und 41a, von denen eines das Aktivierungsglied 31 und das andere ein Effektorglied 41 bildet. Das Effektorglied 41 wird quer zu der Vortriebsrichtung V, im Ausführungsbeispiel rechtwinklig zu der Vortriebsrichtung V, hin und her beweglich von dem Insertionsgehäuse 30 gelagert. Das Gelenk 31a, 41a ist wieder ein Kurvengelenk. Die Führungskurve 31a entspricht der Führungskurve 21a des ersten Ausführungsbeispiels. Das Effektorglied 41 bildet das Eingriffselement 41a, das bei Verlängerung des Inserters an der Führungskurve 31a entlang gleitet und aufgrund des geneigten Verlaufs der Führungskurve 31a bei dem Aufziehen des Inserters eine Querbewegung des Effektorglieds 41 in Richtung auf die zentrale Längsachse des Inserters bewirkt. In dem Gelenk 31a, 41a wird somit die gegen die Vortriebsrichtung V weisende Bewegung, die das Insertergehäuse 30 bei dem Aufziehen relativ zu dem Aktivierungsglied 31 ausführt, in die Querbewegung des Effektorglieds 41 umgewandelt. Dessen Gelenkelement bzw. Eingriffselement 41a ist selbst in der Art einer Führungskurve gebildet, wird hier getriebetechnisch jedoch als Eingriffselement bezeichnet. Das Eingriffselement 41a könnte alternativ auch als beispielsweise einfacher Nocken oder Noppen geformt sein. Ebenso könnte das Eingriffselement 41a als Führungskurve bezeichnet und in einer anderen Modifikation das Gelenkelement 31a als vorragender Nocken oder Noppen geformt werden.

Die Schnittstelle, über welche der Inserter den Insertionskopf aktiviert, ist wieder als reiner Druckkontakt gebildet und besteht zwischen dem Effektorglied 41 und dem Empfangsglied bzw. der beweglichen Griffkomponente 12 des Insertionskopfs. Dieser reine, man könnte auch sagen lose Druckkontakt vereinfacht die Handhabung, da für die Aktivierung keine besondere Gelenkverbindung hergestellt werden muss, es genügt das Aufnehmen des Insertionskopfs in Kombination mit der Betätigung des Aktivierungsglieds 31, was in den Ausführungsbeispielen durch die Aufziehbewegung erfolgt. Der Druckkontakt, d. h. die von dem Effektorglied 41 ausgeübte Druckkraft, wirkt auf die bewegliche Griffkomponente 12 parallel zu der Richtung ihrer Beweglichkeit relativ zu der Basis 1, 2. Durch Zwischenschaltung des Effektorglieds 41 und Verlagerung des Gelenks 31a, 41a gänzlich zum Inserter wird auf die Griffkomponente 12 im zweiten Ausführungsbeispiel vorteilhafterweise quer zu der Richtung der Beweglichkeit der Griffkomponente 12 keine Kraft ausgeübt.

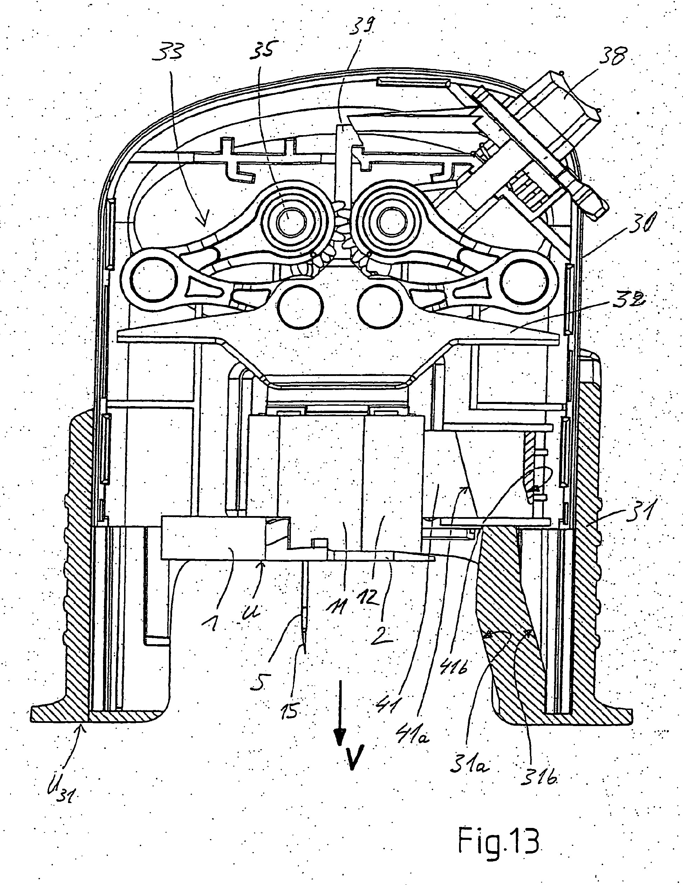

Fig. 13 zeigt das System mit aktiviertem Insertionskopf. Im Verlaufe der Aufziehbewegung des Insertergehäuses 30, was auch als Betätigung des Aktivierungsglieds 31 verstanden wird, wurden die Einführeinrichtung 5 und die Einstecheinrichtung 15 in die Einführposition geschwenkt, so dass ihre gemeinsame Längsachse in die Vortriebsrichtung V weist. Die bewegliche Griffkomponente 12 hat wie zum Insertionskopf beschrieben die Verbindung zwischen dem Griff 10, 12 und der Basis 1, 2 gelöst. Der zwischen der Einführeinrichtung 5 und der Einstecheinrichtung 15 bestehende Reibschluss hält jedoch die Basis 1, 2 wie im ersten Ausführungsbeispiel an dem im Halteeingriff befindlichen Griff 10, 12.

Durch Betätigung des Auslösers 38 wird der Blockiereingriff, in dem sich das Blockierglied 39 noch mit dem Insertergehäuse 30 oder einer damit fest verbundenen Struktur befindet, gelöst und der Krafterzeuger 33 beschleunigt das Vortriebselement 32 in die Vortriebsrichtung V. Die Beschleunigung erfolgt wieder schlagartig, so dass auch die Antriebseinrichtung 32, 33 des zweiten Ausführungsbeispiels in der Art eines Hammers wirkt. Die Antriebskraft wird von zwei Schenkelfedern erzeugt, von denen je eine auf eines der beiden Schenkelpaare wirkt. Die im ortsfesten Drehlager 35 befestigten Schenkel 24 sind über einen Zahneingriff miteinander gekoppelt, der für eine synchrone Ausfahrbewegung der beiden Schenkelpaare sorgt.

Um den Inserter nach Platzierung des Insertionskopfs für eine erneute Verwendung bereitmachen zu können, muss das Effektorglied 41 aus der in Fig. 13 gezeigten Endposition wieder zurück in die in Fig. 12 gezeigte Endposition bewegt werden. Für diese Rückholbewegung bilden das Aktivierungsglied 31 und das Effektorglied 41 ein weiteres Gelenk 31b, 41b, das im Ausführungsbeispiel ebenfalls ein Kurvengelenk ist. Das Aktivierungsglied 31 bildet für das weitere Gelenk die Führungskurve 31b, und das Effektorglied 41 bildet das Eingriffselement 41b. Die Führungskurve 31b verläuft zumindest im Wesentlichen parallel zu der Führungskurve 31a. Die Führungskurven 31a und 31b sind an dem inneren Hülsenteil des Aktivierungsglieds 31 gebildet, die Führungskurve 31a an der Innenfläche und die Führungskurve 31b an der Aussenfläche des inneren Hülsenteils. Sie liegen sich in etwa auf der gleichen Höhe, bezogen auf die Vortriebsrichtung V, gegenüber. Das Eingriffselement 41b liegt dem Eingriffselement 41a ebenfalls gegenüber mit einem lichten Abstand, so dass das innere Hülsenteil des Aktivierungsglieds 31 zwischen den beiden Eingriffselementen 41a und 41b ein- und ausfahren kann.

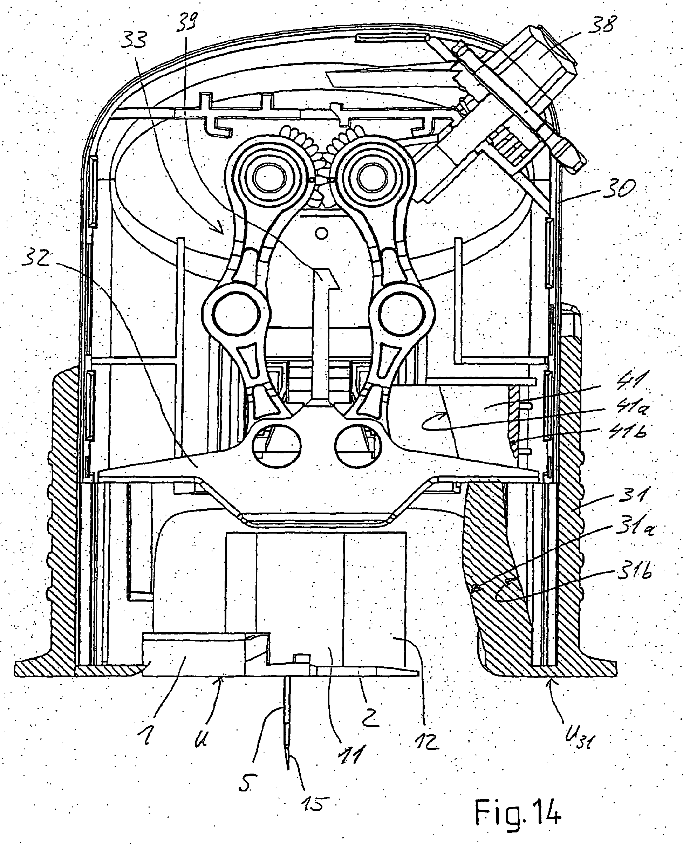

Fig. 14 zeigt das System des zweiten Ausführungsbeispiels mit dem platzierten Insertionskopf. Der Inserter wird von dem Insertionskopf entfernt. Anschliessend zieht der Benutzer den Griff 10, 12 von der Basis 1, 2 ab, wobei die Einstecheinrichtung, gegebenenfalls nach einem erneuten Zusammendrücken der beiden Griffkomponenten 10, 12 in Zusammenschieberichtung und anschliessendes Entlasten derselben, automatisch in eine Schutzposition in den Griff eingeklappt wird, und schliesst den Insertionskopf an einen Katheter einer Infusionspumpe an. In einer auch bereits zum ersten Ausführungsbeispiel erwähnten Modifikation, in welcher die Halteeinrichtung ortsfest mit dem Vortriebselement 32 verbunden ist und demgemäss den Griff 10, 12 noch halten kann, werden der Inserter und damit gemeinsam der noch gehaltene Griff 10, 12 von der Basis entfernt. Anschliessend wird vorzugsweise mittels eines zusätzlichen Abstreifers der Halteeingriff gelöst, gegebenenfalls nach einem vorherigen automatischen oder durch eine zusätzliche Manipulation bewirkten erneuten Zusammendrücken in Zusammenschieberichtung mit anschliessender Entlastung der beiden Griffkomponenten 10, 12, und der Griff 10, 12 mit der Einstecheinrichtung 15 entsorgt.

Um den Inserter für die Verwendung mit einem weiteren Insertionskopf vorzubereiten, schiebt der Benutzer das Insertergehäuse 30 und das Aktivierungsglied 31 wieder zusammen in die eingefahrene Position, wie sie mit eingesetztem Insertionskopf in Fig. 12 dargestellt ist. Bei der Einfahrbewegung fährt das innere Hülsenteil des Aktivierungsglieds 31 zwischen die Eingriffselemente 41a und 41b des Effektorglieds 41. Bei dieser Einfahrbewegung entsteht die weitere Gelenkverbindung zwischen der Führungskurve 31b und dem Eingriffselement 41b. Bei der Einfahrbewegung wird somit in dem Gelenk 31b, 41b das Effektorglied 41 wieder in die in Fig. 12 eingenommene Endposition zurückbewegt, d. h. in Bezug auf die zentrale Längsachse des Inserters quer, vorzugsweise radial, nach auswärts bewegt.

Das unter der Einwirkung der Federeinrichtung 33 in die Vortriebsrichtung V ausgefahrene Vortriebselement 32 liegt einer Stirnseite des inneren Hülsenteils, die von der Unterseite U31 des Aktivierungsglieds 31 abgewandt ist, gegenüber. Durch Anschlagkontakt gegen diese Stirnseite wird die Vortriebsbewegung des Vortriebselements 32 gestoppt. Das Aktivierungsglied 31 ist geometrisch so bemessen, dass es in der ausgefahrenen Position des Teleskops 30, 31 das Vortriebselement 32 genau dann stoppt, wenn die Unterseite U des Insertionskopfs die Höhe der Unterseite U31 erreicht hat und daher bei aufgesetztem Inserter die Hautoberfläche gerade kontaktiert. Bei der Einfahrbewegung des Insertergehäuses 30 relativ zum Aktivierungsglied 31 bzw. des Aktivierungsglieds 31 relativ zu dem Insertergehäuse 30 wird das Vortriebselement 32 wegen des Anschlagkontakts vom Aktivierungsglied 31 gegen die Kraft des Krafterzeugers 33 tiefer in das Insertergehäuse 30 gedrückt, bis das Blockierglied 39 wieder im Blockiereingriff ist, wie ihn beispielhaft die Figuren 13 und 14 zeigen.

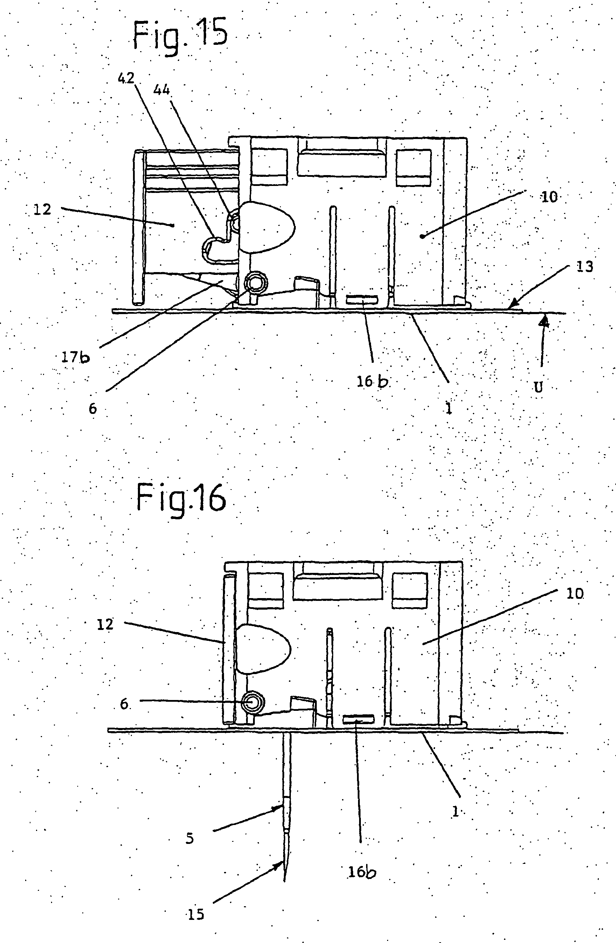

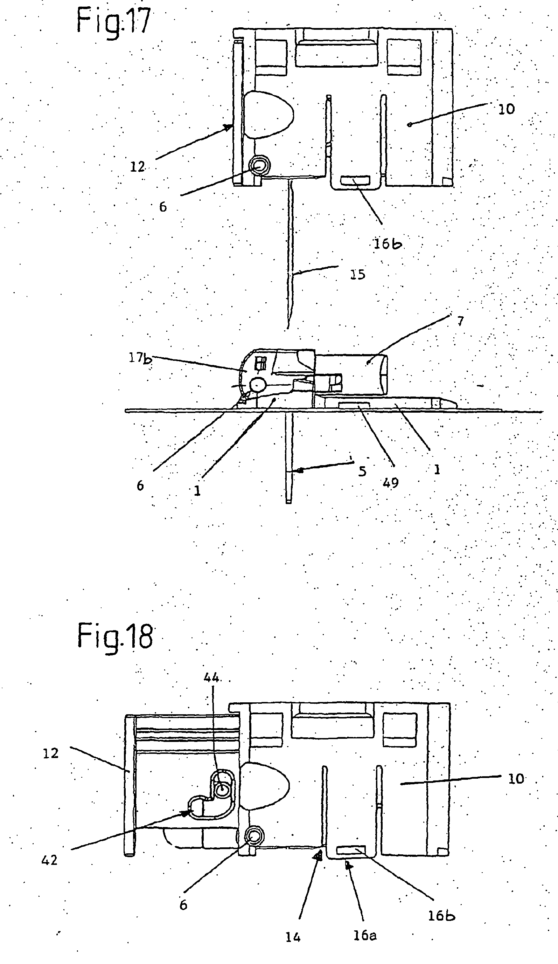

In Fig. 15 ist eine weitere Ausführungsform des erfindungsgemässen Insertionskopfes dargestellt. Der Insertionskopf umfasst eine zweite Griffkomponente 12, die entgegen einer Federkraft in eine erste Griffkomponente 10 eingeschoben werden kann und dann, wie schon beim Insertionskopf gemäss den Figuren 5 bis 9, reversibel über einen sogenannten "Kugelschreibermechanismus" mit dieser verrastet, derart, dass durch ein erneutes Zusammendrücken der beiden Griffkomponenten 10, 12 in Zusammenschieberichtung die Verrastung wieder gelöst wird und die Griffkomponenten 10, 12 durch die Federkraft wieder in die Ursprungsposition auseinander bewegt werden. Durch das Einschieben der zweiten Griffkomponente 12 in die erste Griffkomponente 10 wird ein Kanülengehäuse 17b um ein Gelenk 6 verschwenkt. Das Kanülengehäuse 17b trägt dabei noch in der Schutzposition die Einführeinrichtung 5 und die Einstecheinrichtung 15. Während der Betätigung der zweiten Griffkomponente 12 wird der Kulissenstein 44 in der Kulissenführung 42 im Wesentlichen nach unten in Richtung des Gelenkelementes 6 verfahren, was zu einer Dreh- bzw. Schwenkbewegung für die Einführeinrichtung und die Einstechnadel führt. Sobald der Kulissenstein bzw. der Zapfen 44 im Kurvenbereich der Kulissenführung 42 angelangt ist, ist die Schwenkbewegung von der Schutzposition in die Einführposition vollendet. Die erste Griffkomponente 10 ist noch mit einer Verbindungseinrichtung 16b ausgestattet, die im Eingriff mit einem korrespondierenden Abschnitt 49 (siehe Fig. 17) an der Basis 1 steht, um somit die Basis 1 reversibel mit dem Griff 10, 12 zu verbinden.

Natürlich kann der Kulissenstein auch eine andere Position aufweisen. So könnte der Kulissenstein 44 auch weiter unten angeordnet sein und beim Verschwenken der Bestandteile dann im Wesentlichen nach oben wandern. Hier sind verschiedene Konfigurationen für die Kulissenführung und den Kulissenstein möglich.

Gemäß Fig. 16 ist die zweite Griffkomponente 12 in die erste Griffkomponente 10 eingeschoben und reversibel mit dieser verrastet, wobei die Einführbewegung der zweiten Griffkomponente 12 in die erste Griffkomponente 10 zu dem Herausschwenken der Einführeinrichtung 5 zusammen mit der Einstecheinrichtung 15 aus der Aufnahme 14 (siehe Fig. 18) in die Einführposition geführt hat.

Aus Fig. 17, welche den Griff 10, 12 mit der daran gehaltenen Einstecheinrichtung 15 separiert von dem applizierten Kanülengehäuse 17b mit Basis 1 und Kanüle 5 zeigt, ist ersichtlich, wie der Griff, bestehend aus den Griffkomponente 12 und 10 von der Basis 1 entkoppelbar ist. Die Basis 1 enthält eine Aussparung 49, mit der in der Schutzposition (Fig. 15) eine Verbindungseinrichtung 16b des Griffs 10, 12 im Eingriff ist. Dieser Eingriff wird beim Zusammenschieben der beiden Griffkomponenten 10, 12 aufgehoben, so dass der Griff 10, 12 von der Basis 1 abnehmbar ist.

Die Fig. 18 zeigt den Griff 10, 12 gemäß Fig. 17, wobei hier durch ein erneutes Ineinanderdrücken der beiden Griffkomponenten 10, 12 in Einschieberichtung die Verrastung zwischen diesen beiden Komponenten 10, 12 aufgehoben wurde und diese durch die Kraft einer internen Feder automatisch wieder in die Ursprungsposition auseinandergeschoben worden sind, so dass der Kulissenstein bzw. der Zapfen 44 in der Kulissenführung 42 im Wesentlichen nach oben geführt worden ist, wodurch der Nadelhalter mit der daran festgelegten Einstecheinrichtung 15 wieder verschwenkt worden sind, und zwar in die ursprüngliche Schutzposition in der Aufnahme 14 oder eine dieser nahen Sperrposition.

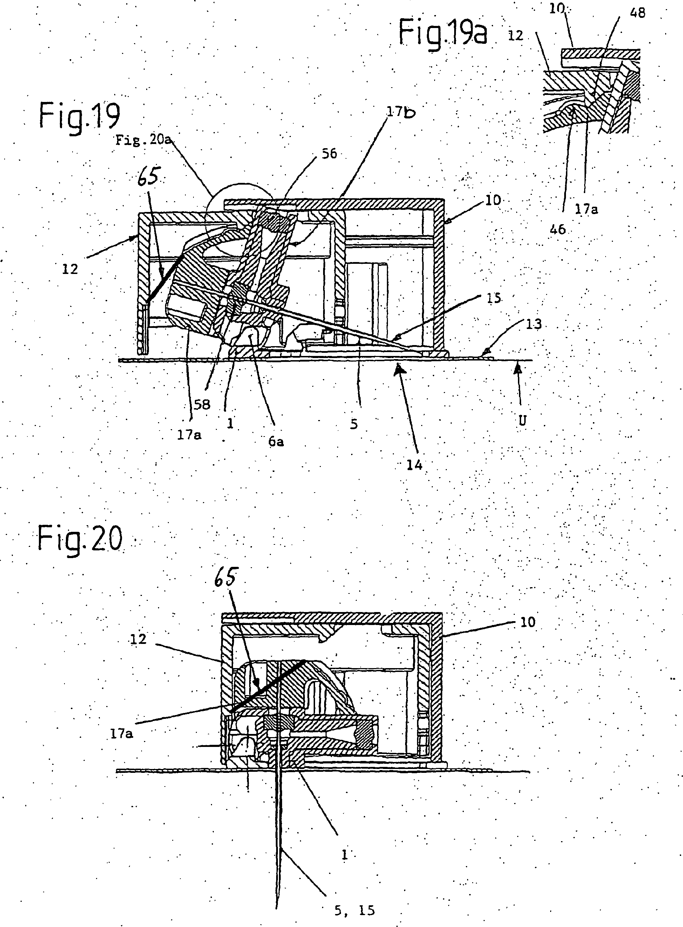

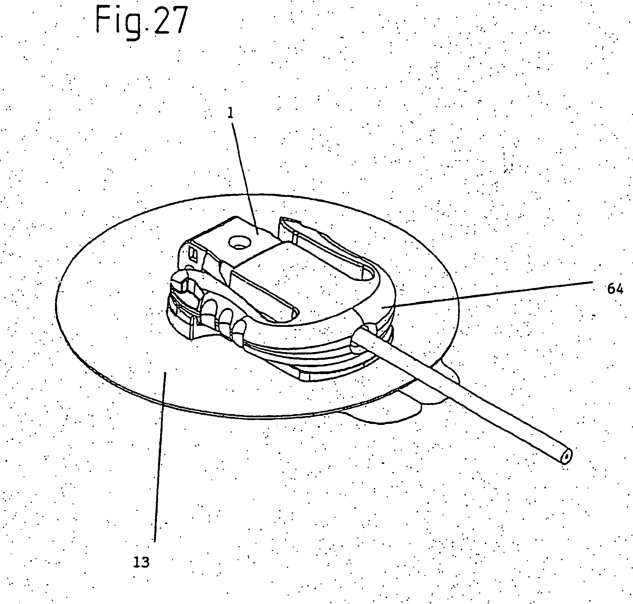

Fig. 19 zeigt eine weitere bevorzugte Ausführungsform des erfindungsgemässen Insertionskopfes in einer Situation vor einer Applikation bzw. während der Lagerung. Die Einführeinrichtung 5 mit der darin teilweise aufgenommenen Einstecheinrichtung 15 befindet sich in der Schutzposition in der Aufnahme 14. Auf der Lasche 13 bzw. dem Pflaster 13 ist eine Schutzfolie auf der Unterseite U aufgebracht, um die Klebestellen des Pflasters 13 aktiv zu halten. Gemäß Fig. 19 ist ferner ein Septum 58 vorgesehen, dass das Einführen und Herausnehmen der Einstecheinrichtung 15 ermöglichen soll, wobei anschliessend eine Abdichtung für die Einführeinrichtung 5 gewährleistet sein soll. Entsprechend ist auch für den senkrecht an die Einstecheinrichtung 15 anschließenden Anschlussabschnitt ein Septum 56 vorgesehen, das einen dichten Anschluss an eine Zufuhrleitung bzw. einen Konvektor (siehe Fig. 27) ermöglichen soll.

An einem auf dem Kanülengehäuse 17b angeordneten Nadelhalter 17a, der die Einstecheinrichtung 15 hält, ist auf der der Basis 1 abgewandten Seite eine Sicherungsstruktur 46 vorgesehen, die sich aus dem Bildausschnitt gemäß Fig. 19a ergibt. Ein Eingriffselement 48 ist im dargestellten Zustand im Eingriff mit der Sicherungsstruktur 46 und ergibt einen Widerstand, der einem zufälligen Einführen der zweiten Griffkomponente 12 in die erste Griffkomponente 10 entgegenwirkt. Der Bediener des erfindungsgemäßen Insertionskopfes gemäß dieser Ausführungsform muss anfangs einen erhöhten Kraftaufwand bereitstellen, um den Eingriff zwischen dem Eingriffselement 48 und der Sicherungsstruktur 46 zu überwinden und um damit den Schwenkvorgang für die Einführeinrichtung 5 nebst Einstecheinrichtung 15 aus der Schutzposition in der Aufnahme 14, wie sie in Fig. 19 dargestellt ist, in die Applikationsposition, wie sie in Fig. 20 dargestellt ist, durchzuführen. Gleichzeitig muss für die Schwenkbewegung zusätzlich die Federkraft zweier als Zugfedern dienender vorgespannter Gummibänder 65 überwunden werden, welche mit zunehmender Schwenkbewegung zunehmend vorgespannt werden.

Auch ist es möglich, die Sicherungsstruktur 46 mit den Elementen 52, 54 entsprechenden Rasteinrichtungen zu realisieren, die beispielsweise an der ersten und der zweiten Griffkomponente 10, 12 vorgesehen sein können und durch das Einschieben der einen in die andere entriegelt werden können.

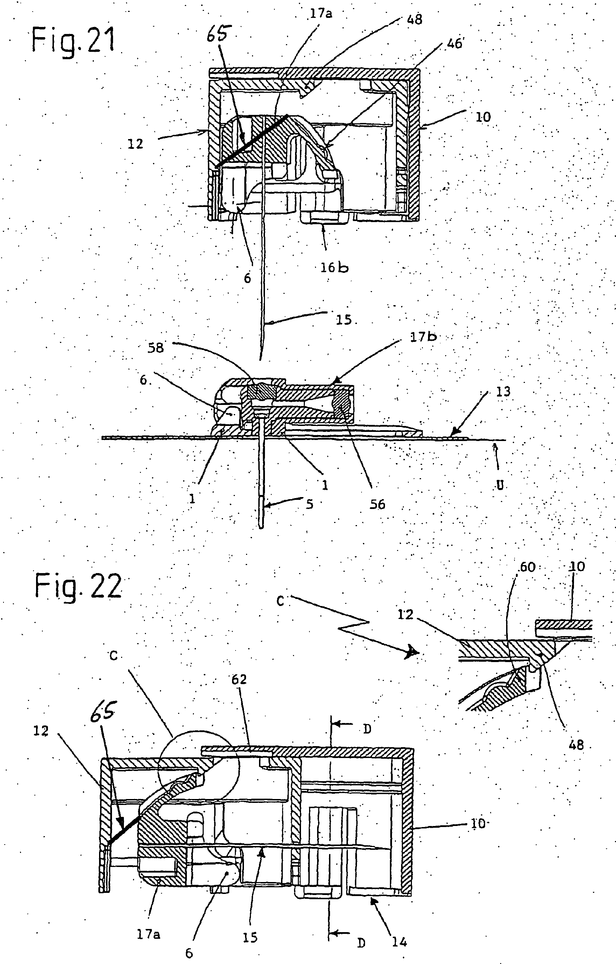

Die in Fig. 20 in das Gewebe eines Patienten eingestochene Einführeinrichtung nebst Einstecheinrichtung 5, 15 ist gemäß Fig. 21 demontiert. D.h., die Einstecheinrichtung bzw. Einstechnadel 15 ist aus der Einführeinrichtung 5 herausgezogen. Das Septum 58 schließt sich und die Einführeinrichtung 5 ist bereit, ein Medikament in den Körper eines Patienten einzuführen.

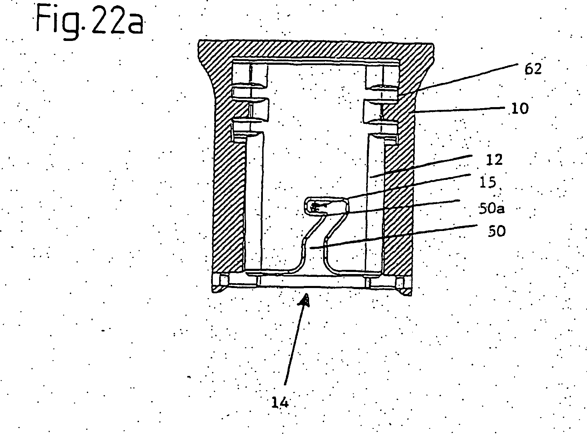

Nach dem Herausziehen der Einstecheinrichtung aus dem Septum 58 kann durch Zurücknahme der auf die beiden Griffkomponenten 10, 12 ausgeübten Druckkraft eine automatische Rückstellbewegung der Einstecheinrichtung 15 durch die Kraft der beiden Gummibänder 70 in eine Schutzposition bewirkt werden. Diese Situation ist in Fig. 22 dargestellt. Dabei wird die Einstecheinrichtung 15 über die ursprüngliche Schutzposition in eine Position oberhalb der Schutzposition, d.h. oberhalb einer nachfolgend erläuterten Rastschulter 60, bewegt. In dieser Position wird die Einstecheinrichtung 15, wie in dem Vergrösserungsbild C erkennbar, dadurch gesichert, dass eine Rastschulter 60 am Ende des Nadelhalters 17a in Eingriff mit dem Eingriffselement 48 gelangt ist. In dieser Position ist es nun nicht mehr möglich, die kontaminierte Einstecheinrichtung 15 wieder aus dem Griff 10, 12 auszulenken, weil eine Sperrwirkung durch das Eingriffselement 48 in Verbindung mit der Rastschulter 60 bereitgestellt wird. Die Einstecheinrichtung 15 wird hinter der Rastschulter in einer Sperrposition gehalten. Gemäß Fig. 22a wird ein Schnitt D-D gemäß Fig. 22 wiedergegeben, um eine Sicherungskulisse 50 darzustellen, die dazu dient, die benutzte bzw. kontaminierte Einstecheinrichtung 15 in einen gesicherten Kulissenabschnitt hinter einer Sicherungsschulter 50a zu überführen. Demgegenüber kann die ungenutzte, sterile Einstecheinrichtung vor der Benutzung in einer anderen Position vor der Sicherungsschulter 50a gehalten werden.

In der Schnittdarstellung gemäß Fig. 22a sind auch Führungen 62 zu erkennen, die eine Führung der zweiten Griffkomponente innerhalb der ersten Griffkomponente 10 ermöglichen. Wird die Einstecheinrichtung 15 in eine inaktive Position in der Aufnahme 14 zurückgeschwenkt, wird sie sich, sich flexibel verformend, durch die Sicherungskulisse 50 bewegen, um anschließend im oberen Bereich der Sicherungskulisse 50 hinter der Sicherungsschulter 50a entlastet zu werden und in eine unmittelbar in Fig. 22a dargestellte Position zurückschnellen, um dann sicher in der dargestellten Position zu verharren und dort blockiert zu sein.

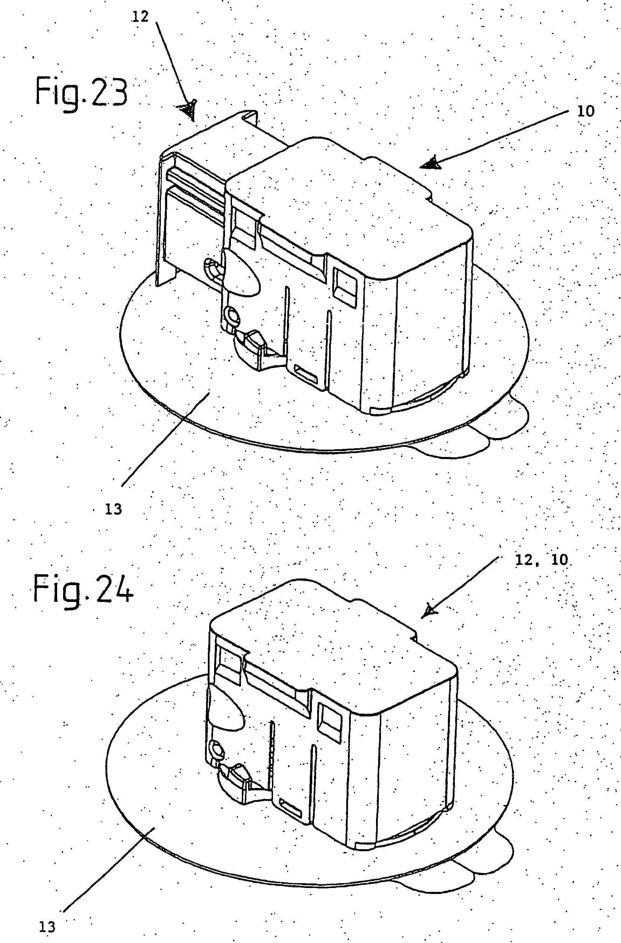

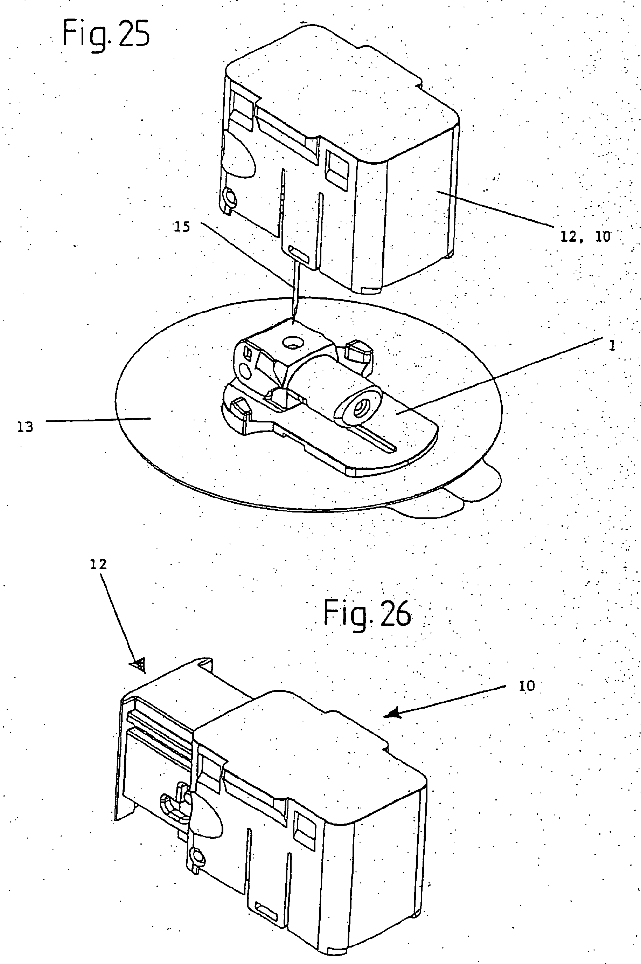

Die Figuren 23 bis 28 zeigen verschiedene Stadien der Applikation eines erfindungsgemäßen Insertionskopfes mit einer ähnlichen Funktionsweise wie der zuvor diskutierte. Hier werden jedoch die beiden Griffkomponenten 10, 12 beim ersten Zusammenschieben, welches dem Ausklappen der Einführeinrichtung 5 und der Einstecheinrichtung 15 von der Schutzposition in die Einführposition dient, über einen so genannten "Kugelschreibermechanismus" miteinander verrastet, derart, dass diese Verrastung durch ein erneuten Aufeinanderzubewegen der beiden Griffkomponenten 10, 12 in Zusammenschieberichtung wieder aufgehoben werden kann. Wie ein solcher "Kugelschreibermechanismus" realisiert werden kann, wurde bereits zuvor anhand der Figuren 5 bis 9 dargelegt und ist dem Fachmann auch geläufig, so dass an dieser Stelle nicht weiter darauf eingegangen werden muss. Gemäß Fig. 23 ist die zweite Griffkomponente 12 in einer aus der ersten Griffkomponente 10 herausragenden Position dargestellt, in der die Einführeinrichtung 5 nebst Einstechnadel 15 in ihrer Schutzposition ist. Bei der Darstellung gemäß Fig. 24 ist die zweite Griffkomponente 12 in die erste Griffkomponente 10 hinein bewegt und mit dieser verrastet worden, so dass, wie sich aus Fig. 25 ergibt, die Einführeinrichtung und die Einstecheinrichtung in den Körper eines Patienten eingestochen werden können.

Die Fig. 25 zeigt nun den Zustand, nachdem die Einführeinrichtung nebst Einstecheinrichtung 15 in dem Körper eines Patienten eingestochen und die Einstecheinrichtung 15 mit dem Griff 10, 12 von der Basis 1 mit der Einführeinrichtung separiert worden ist.

Durch ein erneutes Ineinanderdrücken der beiden Griffkomponenten 10, 12 in der in Fig. 25 dargestellten Situation wird die Verrastung der beiden Griffteile 10, 12 aufgehoben, so dass die zweite Griffkomponente 12 durch die Kraft der internen Feder wieder aus der ersten Griffkomponente 10 herausgedrückt wird, unter einem Überführen der Einstecheinrichtung 15 in eine Schutzposition innerhalb der von den Griffkomponenten 10, 12 gebildeten Begrenzungen.

Wie in Fig. 27 gezeigt kann nun ein Konnektor 64 mit einer Zuführleitung an die Basis angedockt werden, um ein Medikament, beispielsweise Insulin, zuzuführen.

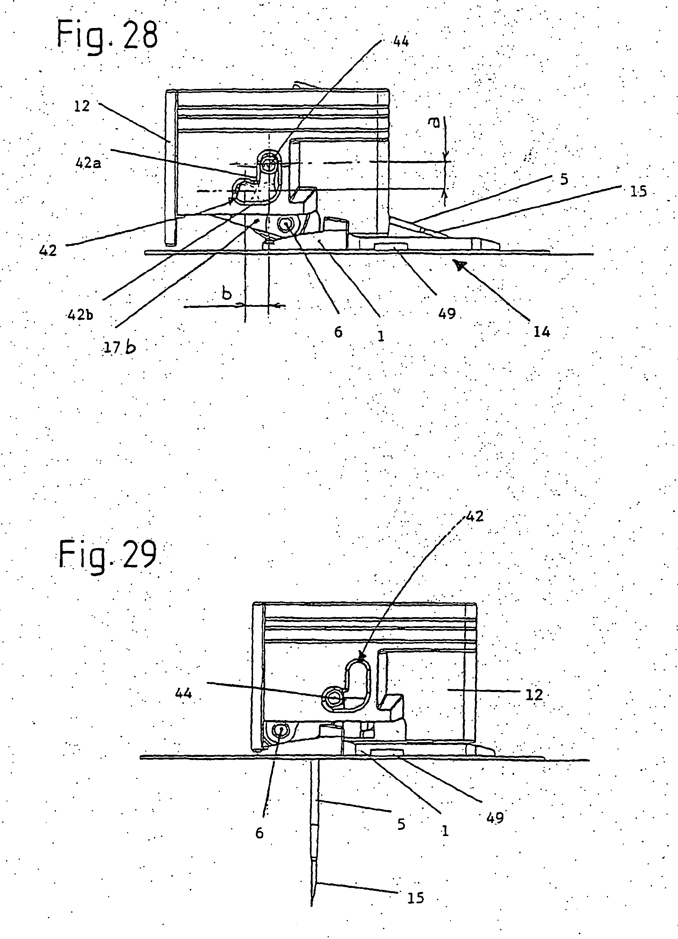

In der Fig. 28 ist dargestellt, dass die Führungskulisse 42 einen ersten Abschnitt 42a der Länge a hat.. Diese Strecke a dient dazu, das Kanülengehäuse 17b und damit die Einführeinrichtung 5 nebst der Einstecheinrichtung 15 durch Betätigen bzw. Vorschub der zweiten Griffkomponente 12 im dargestellten Falle nach rechts zu verschwenken, so dass der Kulissenstein 44 sich im Wesentlichen nach unten in Richtung des Gelenkelementes 6 bewegt, während die Einführeinrichtung 5 aus der Schutzposition in die Applikationsposition bzw. Einführposition überführt wird. Ein zweiter Abschnitt 42b der Kulissenführung 42 steht zur Verfügung, um über die zweite Strecke b einen Spielraum zu verschaffen, um eine Entriegelung vornehmen zu können, um die Verbindungseinrichtung 16b, aus der Eingriffsausnehmung 49 herauslenken zu können.

Dementsprechend ist gemäß Fig. 29, in der der Kulissenstein 44 am anderen Ende der Kulissenführung 42 relativ zu der Darstellung gemäß Fig. 28 angelangt ist, die Einführeinrichtung 5 nebst Einstecheinrichtung 15 nicht nur in ihrer Applikationsposition, sondern der Griff 10, 12 ist zudem gegenüber der Basis 1 bereits entriegelt und kann abgenommen werden, wobei die Einstecheinrichtung 15 aus der Einführeinrichtung 5 herausgezogen wird.

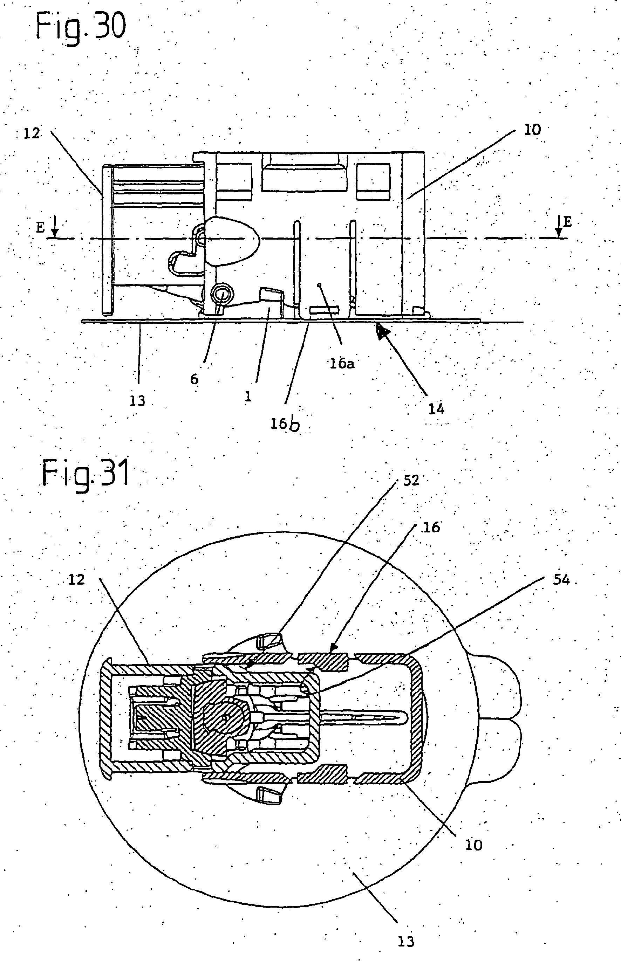

Bei der Ausführungsform gemäß Fig. 30 ist zusätzlich ein Entriegelungsmechanismus dargestellt, der sich insbesondere im Schnitt E-E ergibt, welcher in Fig. 31 wiedergegeben ist. Wie zu erkennen ist, weist die zweite Griffkomponente 12 eine Steuerrampe 52 auf, die nach dem Einführen der zweiten Griffkomponente 12 in die erste Griffkomponente 10 gegen eine Auslenkrampe 54 anläuft, um auf diese Weise das Verbindungselement 16 aus seinem Eingriff mit der Eingriffsausnehmung 49 an der Basis 1 herauszubefördern. Während die Bewegung zum Verschwenken der Einführeinrichtung 5 nebst Einstecheinrichtung 15 der Strecke 42a mit der Länge a entspricht, entspricht die Bewegung zum Führen der Steuerrampe 52 gegen die Auslenkrampe 54 dem Abschnitt 42b der Führungskulisse 42 gemäß Fig. 28.

### Bezugszeichen:

- 1: Basis, Aufnahme
- 2: Basis, Flachteil
- 3: Ausnehmung
- 4: Ausnehmung
- 5: Einführeinrichtung
- 6: Gelenkelement
- 6a: Gelenkelementgegenstück
- 7: Zuführung
- 8: Führungsnut
- 9: Verbindungselement
- 10: Erste Griffkomponente
- 11: Grifffläche
- 12: Zweite Griffkomponente, Empfangsglied
- 12a Gelenkelement,: Eingriffselement, Druckkontaktfläche
- 13: Lasche, Pflaster
- 14: Aufnahme
- 15: Einstecheinrichtung
- 16: Verbindungselement
- 16a: Federsteg
- 16b: Verbindungseinrichtung
- 17: federelastischer Arm
- 17a: Nadelhalter, Halteelement
- 17b: Kanülengehäuse
- 18: Führungszylinder
- 19: Verbindungselement
- 20: Insertergehäuse
- 21: Aktivierungsglied
- 21a: Gelenkelement, Führungskurve
- 22: Vortriebselement
- 23: Krafterzeuger
- 24: Schenkel
- 25: Drehgelenk
- 26: Drehgelenk
- 27: Drehgelenk
- 28: Auslöser
- 29: Blockierglied
- 30: Insertergehäuse
- 31: Aktivierungsglied
- 31a: Gelenkelement, Führungskurve
- 31b: Gelenkelement, Führungskurve
- 32: Vortriebselement
- 33: Krafterzeuger
- 34: Schenkel
- 35: Drehgelenk
- 36: Drehgelenk
- 37: Drehgelenk
- 38: Auslöser
- 39: Blockierglied
- 40: Wellenstumpf
- 41: Effektorglied
- 41a: Gelenkelement, Eingriffselement
- 41b: Gelenkelement, Eingriffselement
- 42: Kulissenführung
- 42a: Schwenkführungsabschnitt
- 42b: Entrastungsführungsabschnitt
- 44: Kulissenstein, Zapfen
- 46: Sicherungsstruktur
- 48: Eingriffselement
- 49: Eingriffsausnehmung
- 50: Sicherungskulisse
- 50a: Sicherungsschulter
- 52: Steuerrampe
- 54: Auslenkrampe
- 56: Septum
- 58: Septum
- 60: Rastschulter
- 62: Führung
- 64: Konnektor
- 65: Zugfeder
- 66: Druckfeder

- U: Unterseite
- V: Vortriebsrichtung

## Patentansprüche

1. Insertionskopf für medizinische oder pharmazeutische Anwendungen, umfassend:
a) ein Gehäuse (10, 12) mit einem ersten Gehäuseteil (10) und einem zweiten Gehäuseteil (12), wobei der erste und der zweite Gehäuseteil relativ zueinander bewegbar sind;
b) eine Basis (1, 2);
c) eine flexible Einführeinrichtung (5), welche von einer Einstecheinrichtung (15) stabilisiert ist, wobei die Einführeinrichtung (5) und die Einstecheinrichtung (15) gemeinsam bewegbar gelagert sind,
d) wobei die Einführeinrichtung (5) und die Einstecheinrichtung (15) relativ zum Gehäuse (10, 12) aus einer Schutzposition, in der die freien Enden der Einführeinrichtung (5) und der Einstecheinrichtung (15) innerhalb der Begrenzungen des Gehäuses (10, 12) und/oder der Basis (1, 2) angeordnet sind, in eine Einführposition bewegbar sind, in der die freien Enden aus den Begrenzungen des Gehäuses (10, 12) und/oder der Basis (1, 2) hervorragen, derart, dass diese in ein organisches Gewebe eingeführt werden können, und
e) eine Kopplung (17a, 40, 42, 44), welche die Relativbewegung der beiden Gehäuseteile (10, 12) zueinander in eine Bewegung der Einführeinrichtung (5) und der Einstecheinrichtung (15) überträgt, derart, dass diese durch Bewegen der beiden Gehäuseteile (10, 12) relativ zueinander von der Schutzposition in die Einführposition bewegbar sind,
wobei die Basis (1, 2) in der Einführposition eine auf dem organischen Gewebe platzierbare Unterseite (U) aufweist, von welcher die Einführeinrichtung (5) und die Einstecheinrichtung (15) hervorstehen, zur Ermöglichung eines Einführens derselben in das Gewebe unter einem Platzieren der Basis (1, 2) mit der Unterseite (U) auf dem Gewebe, wobei in der Einführposition die Einstecheinrichtung (15) derartig mit dem Gehäuse (10, 12) verbunden ist und von der Einführeinrichtung (5) lösbar ist, dass die Einstecheinrichtung (15) nach dem Einführen der Einführeinrichtung (5) und der Einstecheinrichtung (15) in das Gewebe durch Ergreifen des Gehäuses (10, 12) mit einer Hand und Bewegen desselben mit der Hand in einer Richtung entgegengesetzt der bestimmungsgemässen Einsetzrichtung von der Basis (1, 2) und der Einführeinrichtung (5) entfernt werden kann, wobei Rückstellmittel (65, 66) vorhanden sind, mittels welcher die Einstecheinrichtung (15) direkt nach dem Entfernen von der Basis (1, 2) und der Einführeinrichtung (5) automatisch zurück in eine Schutzposition, in welcher das freie Ende der Einstecheinrichtung (15) innerhalb der Begrenzungen des Gehäuses (10, 12) angeordnet ist, bewegbar ist, gegebenenfalls nach Betätigung eines Auslöseorgans mit der das Gehäuse (10, 12) haltenden Hand.

2. Insertionskopf nach Anspruch 1, **dadurch gekennzeichnet, dass** die beiden Gehäuseteile (10, 12) zwischen zwei Fingern einer Hand greifbar sind, wobei mit je einem der Finger gegen eines der Gehäuseteile (10, 12) drückend das zweite Gehäuseteil (12) in Richtung auf das erste Gehäuseteil (10) zu bewegbar ist.

3. Insertionskopf nach einem der vorangehenden Ansprüche, **dadurch gekennzeichnet, dass** die Basis (1, 2) unbeweglich mit der Einführeinrichtung (5) verbunden ist, so dass sie beim Bewegen derselben von der Schutzposition in die Einführposition gegenüber dem Gehäuse (10, 12) bewegt wird.

4. Insertionskopf nach einem der Ansprüche 1 bis 2, **dadurch gekennzeichnet, dass** die Unterseite (U) der Basis (1, 2) bei in der Schutzposition befindlicher Einführ- (5) und Einstecheinrichtung (15) zumindest einen Teil einer Aussenseite des Gehäuses (10, 12) bildet, so dass deren freie Enden in der Schutzposition hinter der Unterseite (U) der Basis (1, 2) zurückstehen, wobei die Einführeinrichtung (5) und die Einstecheinrichtung (15) durch Bewegen derselben von der Schutzposition in die Einführposition gegenüber der Basis (1, 2) bewegbar sind, derart, dass deren freie Enden in der Einführposition über die Unterseite (U) der Basis (1, 2) vorragen.

5. Insertionskopf nach Anspruch 4, **dadurch gekennzeichnet, dass** die Einführeinrichtung (5) in der Basis (1, 2) beweglich gelagert ist.

6. Insertionskopf nach Anspruch 5, **dadurch gekennzeichnet, dass** die Basis (1, 2) und ein Gelenkelement (6) oder ein Führungselement einer Schiebeführung miteinander ein Gelenk oder eine Schiebeführung bilden und die Einführeinrichtung (5) von dem Gelenkelement (6) oder dem Führungselement abragt.

7. Insertionskopf nach einem der Ansprüche 4 bis 6, **dadurch gekennzeichnet, dass** das Gehäuse (10, 12) von einer Oberseite der Basis (1, 2) aufragt.

8. Insertionskopf nach einem der vorangehenden Ansprüche, **dadurch gekennzeichnet, dass** die beiden Gehäuseteile (10, 12) relativ zueinander schwenkbar oder verschiebbar, insbesondere ineinander schiebbar sind, zur Bewirkung der Bewegung der Einführeinrichtung (5) und der Einstecheinrichtung (15) von der Schutzposition in die Einführposition.

9. Insertionskopf nach Anspruch 8, **dadurch gekennzeichnet, dass** die beiden Gehäuseteile (10, 12) linear zueinander verschiebbar sind, zur Bewirkung der Bewegung der Einführeinrichtung (5) und der Einstecheinrichtung (15) von der Schutzposition in die Einführposition..

10. Insertionskopf nach Anspruch 4 und nach Anspruch 9, **dadurch gekennzeichnet, dass** die beiden Gehäuseteile (10, 12) im Wesentlichen parallel zur Unterseite (U) der Basis (1, 2) verschiebbar sind.

11. Insertionskopf nach einem der vorhergehenden Ansprüche, **dadurch gekennzeichnet, dass** die Einführeinrichtung (5) in die Einführposition schwenkbar ist.

12. Insertionskopf nach einem der vorangehenden Ansprüche, **dadurch gekennzeichnet, dass** die Kopplung (17a, 40, 42, 44) die beiden Gehäuseteile (10, 12), insbesondere eines der beiden Gehäuseteile (12), mittels Zahneingriffs, vorzugsweise mittels eines Zahnrads und einer Zahnstange, mit der Einführeinrichtung (5) koppelt.

13. Insertionskopf nach Anspruch 12, **dadurch gekennzeichnet, dass** eines der beiden Gehäuseteile (12) mit einer Zahnstange, die vorzugsweise an diesem Gehäuseteil (12) geformt ist, und die Einführeinrichtung (5) mit einem mit der Zahnstange in dem Zahneingriff befindlichen Zahnrad verbunden ist.

14. Insertionskopf nach Anspruch 11 und nach einem der Ansprüche 12 bis 13, **dadurch gekennzeichnet, dass** die Einführeinrichtung (5) um eine Rotationsachse schwenkbar und drehsteif mit einem um die Rotationsachse drehbaren, in dem Zahneingriff befindlichen Zahnrad verbunden ist.

15. Insertionskopf nach einem der Ansprüche 1 bis 11, **dadurch gekennzeichnet, dass** die Einführeinrichtung (5) und/oder die Einstecheinrichtung (15) über eine Drehachse (6, 40) an der Basis (1) oder an einem ersten der beiden Gehäuseteile (10) gehalten ist, ein Kulissenstein (44) vorhanden ist, der in Bezug auf die Drehachse (6, 40) exzentrisch angeordnet ist, eine Kulissenführung (42) vorhanden ist, die im Wirkzusammenhang mit dem Kulissenstein (44) steht, derart, dass eine Bewegung der beiden Gehäuseteile (10, 12) relativ zueinander den Kulissenstein (44), geführt durch die Kulissenführung (42), aus einer ersten Stellung, die der Schutzposition der Einführeinrichtung (5) und der Einstecheinrichtung (15) entspricht, in eine zweite Stellung, die der Einführposition der Einführeinrichtung (5) und der Einstecheinrichtung entspricht, überführt.

16. Insertionskopf nach einem der vorangehenden Ansprüche, **dadurch gekennzeichnet, dass** das Gehäuse (10, 12) mit der Basis (1, 2) lösbar verbunden ist und insbesondere, dass sich diese Verbindung bei der Bewegung der beiden Gehäuseteile (10, 12) zum Bewegen der Einführeinrichtung (5) und der Einstecheinrichtung (15) von der Schutzposition in die Einführposition löst oder bei einer auf diese Bewegung folgenden weiteren Bewegung der beiden Gehäuseteile (10, 12) relativ zueinander.

17. Insertionskopf nach Anspruch 16, **dadurch gekennzeichnet, dass** an der Basis (1, 2) ein erstes Verbindungselement (49) und am Gehäuse (10, 12) ein zweites Verbindungselement (16b) geformt ist, dass die Verbindungselemente (49, 16b) miteinander in einem die Verbindung schaffenden Eingriff sind und dass wenigstens eines der Verbindungselemente (16b) gegen eine Elastizitätskraft aus dem Eingriff bewegbar ist.

18. Insertionskopf nach einem der Ansprüche 16 bis 17, **dadurch gekennzeichnet, dass** ein erstes Verbindungselement (49) und ein zweites Verbindungselement (16b) miteinander in einem die Verbindung schaffenden Eingriff sind und eines der beiden Gehäuseteile (12) bei seiner Bewegung eines der Verbindungselemente (16b) kontaktiert und gegen eine Elastizitätskraft aus dem Eingriff bewegt.

19. Insertionskopf nach Anspruch 18, **dadurch gekennzeichnet, dass** eines der beiden Gehäuseteile (12) soweit in das andere der beiden Gehäuseteile (10) einschiebbar ist, dass eine Steuerrampe (52) an dem ersten Gehäuseteil (12) nach Beendigung des Vorgangs zur Überführung der Einführeinrichtung (5) und der Einstecheinrichtung (15) von der Schutzposition in die Einführposition gegen eine Auslenkrampe (54) an einem Verbindungselement (16) anstößt, welches die Basis (1, 2) reversibel mit dem Gehäuse (10, 12) verbindet, und dieses aus einer Eingriffsstellung auslenkt, so dass das Gehäuse (10, 12) und die Basis (1, 2) trennbar sind.

20. Insertionskopf nach Anspruch 15 und nach einem der Ansprüche 16 bis 19, wobei der Insertionskopf derartig ausgestaltet ist, dass eine Bewegung der beiden Gehäuseteile (10, 12) relativ zueinander über eine erste Strecke (a) eines ersten Abschnitts (42a) der Kulissenführung (42) den Kulissenstein (44), geführt durch die Kulissenführung (42), aus einer ersten Stellung, die der Schutzposition der Einführeinrichtung (5) und der Einstecheinrichtung (15) entspricht, in eine zweite Stellung, die der Einführposition der Einführeinrichtung (5) und der Einstecheinrichtung (15) entspricht, überführt, wobei eine Verbindungseinrichtung (16b) vorgesehen ist, die die Basis (1, 2) reversibel mit dem Gehäuse (10, 12) verbindet, und wobei zum Lösen des Gehäuses (10, 12) von der Basis (1, 2) insbesondere genau eines der beiden Gehäuseteile (12) über eine zweite Strecke (b) senkrecht zur Erstreckungsrichtung der in der Einführposition befindlichen Einführeinrichtung (5) gegenüber der Basis (1, 2) bewegbar ist, wobei die Kulissenführung (42) einen zu der zweiten Strecke (b) korrespondierenden zweiten Abschnitt (42b) aufweist.

21. Insertionskopf nach einem der vorangehenden Ansprüche, **dadurch gekennzeichnet, dass** die Einstecheinrichtung (15) sich bei der Bewegung der Einführenrichtung (5) und der Einstecheinrichtung (15) in die Einführposition mit dem Gehäuse (10, 12) verbindet.

22. Insertionskopf nach einem der vorangehenden Ansprüche, **dadurch gekennzeichnet, dass** die Basis (1, 2) eine Aufnahme bildet, welche die Einführeinrichtung (5) und die Einstecheinrichtung (15) in deren Schutzposition aufnimmt.

23. Insertionskopf nach einem der vorangehenden Ansprüche, **dadurch gekennzeichnet, dass** das Gehäuse(10, 12) eine Aufnahme (14) bildet, welche die Einführeinrichtung (5) und die Einstecheinrichtung (15) in deren Schutzposition aufnimmt.

24. Insertionskopf nach einem der vorangehenden Ansprüche, **dadurch gekennzeichnet, dass** der Einführeinrichtung (5) eine Sicherungsstruktur zugeordnet ist, welche die Einführeinrichtung (5) bzw. die Einstecheinrichtung (15) in der Schutzposition reversibel sichert.

25. Insertionskopf nach Anspruch 24, **dadurch gekennzeichnet, dass** der Einführeinrichtung (5) ein Kanü-Iengehäuse (17b) zugeordnet ist, welches mit der Einführeinrichtung (5) mitbewegt wird, wobei das Kanülengehäuse (17b) eine Sicherungsstruktur (46) umfasst, die zu einer parallel zu der Bewegungsrichtung der beiden Gehäuseteile (10, 12) relativ zueinander erstreckten Wand eines der Gehäuseteile (12) gegenüberliegt, wobei ein Eingriffselement (48) an der Innenseite dieser Wand mit der Sicherungsstruktur (46) in der Schutzposition der Einführeinrichtung (5) im reversiblen Eingriff steht.

26. Insertionskopf nach einem der vorangehenden Ansprüche, **dadurch gekennzeichnet, dass** in einem der beiden Gehäuseteile (12) und/oder in dem anderen der beiden Gehäuseteile (10), welches die Einführ- (5) und Einstecheinrichtung (15) in der Schutzposition aufnimmt, eine Sicherungskulisse (50) vorgesehen ist, in welche die Einführ- (5) und Einstecheinrichtung (15) in der Schutzposition einrastbar ist.

27. Insertionskopf nach einem der vorangehenden Ansprüche, **dadurch gekennzeichnet, dass** der Einstecheinrichtung (15) ein Halteelement (17a) zugeordnet ist, das bei dem automatischen Zurückbewegen der Einstecheinrichtung (15) in eine Schutzposition mit der Einstecheinrichtung (15) mitbewegt wird, wobei das Halteelement (17a) eine Rastschulter (60) aufweist, die bei dem automatischen Zurückbewegen im Gehäuse (10, 12) in einen verrastenden Eingriff mit einem Eingriffelement (48) tritt, um ein neuerliches Herausbewegen der Einstecheinrichtung (15) aus dem Gehäuse (10, 12) zu verhindern.

## Claims

1. Insertion head for medical or pharmaceutical applications, comprising:
a) a housing (10, 12) with a first housing part (10) and a second housing part (12), wherein the first and second housing parts are movable relative to each other;
b) a base (1, 2);
c) a flexible insertion device (5), which is stabilized by a puncture device (15), wherein the insertion device (5) and the puncture device (15) are mounted so as to be jointly movable,
d) wherein the insertion device (5) and the puncture device (15) are movable relative to the housing (10, 12) from a protected position, in which the free ends of the insertion device (5) and of the puncture device (15) are arranged inside the boundaries of the housing (10, 12) and/or of the base (1, 2), to an insertion position in which the free ends protrude from the boundaries of the housing (10, 12) and/or the base (1, 2) in such a way that they can be inserted into an organic tissue, and
e) a coupling (17a, 40, 42, 44), by means of which the relative movement of the two housing parts (10, 12) relative to each other is converted into a movement of the insertion device (5) and of the puncture device (15) in such a way that these are movable from the protected position to the insertion position by moving the two housing parts (10, 12) relative to each other,
wherein the base (1, 2) in the insertion position, has an underside (U) which can be placed on the organic tissue, from which the insertion device (5) and the puncture device (15) protrude, to permit an insertion thereof into the tissue when the base (1, 2) is placed with the underside (U) on the tissue, wherein the puncture device (15), in the insertion position, is connected to the housing (10, 12) and is releasable from the insertion device (5) such that, after the insertion of the insertion device (5) and of the puncture device (15) into the tissue, it is possible, by gripping the housing (10, 12) with one hand and moving it with the hand in a direction counter to the intended direction of insertion, to remove the puncture device (15) from the base (1, 2) and from the insertion device (5), wherein restoring means (65, 66) are provided by means of which, directly after the removal from the base (1, 2) and from the insertion device (5), the puncture device (15) can be moved back automatically to a protected position in which the free end of the puncture device (15) is arranged inside the boundaries of the housing (10, 12), if appropriate after actuating a trigger member using the hand that is holding the housing (10, 12).

2. Insertion head according to claim 1, **characterized in that** the two housing parts (10, 12) can be gripped between two fingers of one hand, wherein with one finger pressing against one of the housing parts (10, 12), the second housing part (12) is movable in the direction towards the first housing part (10).

3. Insertion head according to one of the preceding claims, **characterized in that** the base (1, 2) is connected immovably to the insertion device (5), such that, upon movement of the latter from the protected position to the insertion position, it is moved relative to the housing (10, 12).

4. Insertion head according to one of claims 1 and 2, **characterized in that**, with the insertion device (5) and puncture device (15) located in the protected position, the underside (U) of the base (1, 2) forms at least a part of an outer face of the housing (10, 12), such that, in the protected position, their free ends are set back behind the underside (U) of the base (1, 2), wherein upon movement from the protected position to the insertion position, the insertion device (5) and the puncture device (15) are movable relative to the base (1, 2) in such a way that their free ends protrude above the underside (U) of the base (1, 2) in the insertion position.

5. Insertion head according to claim 4, **characterized in that** the insertion device (5) is mounted movably in the base (1, 2).

6. Insertion head according to claim 5, **characterized in that** the base (1, 2) and a hinge element (6) or a guide element of a sliding guide together form a hinge or a sliding guide, and the insertion device (5) protrudes down from the hinge element (6) or the guide element.

7. Insertion head according to one of claims 4 to 6, **characterized in that** the housing (10, 12) extends upwards from a top face of the base (1, 2).

8. Insertion head according to one of the preceding claims, **characterized in that** the two housing parts (10, 12) are pivotable or displaceable relative to each other, in particular slidable one inside the other, to effect the movement of the insertion device (5) and of the puncture device (15) from the protected position to the insertion position.

9. Insertion head according to claim 8, **characterized in that** the two housing parts (10, 12) are linearly displaceable relative to each other in order to effect the movement of the insertion device (5) and of the puncture device (15) from the protected position to the insertion position.

10. Insertion head according to claim 4 and claim 9, **characterized in that** the two housing parts (10, 12) are displaceable substantially parallel to the underside (U) of the base (1, 2).

11. Insertion head according to one of the preceding claims, **characterized in that** the insertion device (5) can be pivoted into the insertion position.

12. Insertion head according to one of the preceding claims, **characterized in that** the coupling (17a, 40, 42, 44) couples the two housing parts (10, 12), in particular one of the two housing parts (12), to the insertion device (5) by means of a toothed engagement, preferably by means of a toothed wheel and a toothed rack.

13. Insertion head according to claim 12, **characterized in that** one of the two housing parts (12) is connected to a toothed rack, which is preferably formed on this housing part (12), and the insertion device (5) is connected to a toothed wheel which is in the toothed engagement with the toothed rack.

14. Insertion head according to claim 11 and according to one of claims 12 to 13, **characterized in that** the insertion device (5) is pivotable about a rotation axis and is connected in a rotationally fixed manner to a toothed wheel which rotates about the rotation axis and is in toothed engagement.

15. Insertion head according to one of claims 1 to 11, **characterized in that** the insertion device (5) and/or the puncture device (15) is held via a rotation shaft (6, 40) at the base (1) or at a first of the two housing parts (10), a sliding block (44) is provided which is arranged eccentrically with respect to the rotation shaft (6, 40), a guide slot (42) is provided which is operatively engaged with the sliding block (44) in such a way that a movement of the two housing parts (10, 12) relative to each other transfers the sliding block (44), guided by the guide slot (42), from a first position, which corresponds to the protected position of the insertion device (5) and of the puncture device (15), to a second position, which corresponds to the insertion position of the insertion device (5) and of the puncture device.

16. Insertion head according to one of the preceding claims, **characterized in that** the housing (10, 12) is detachably connected to the base (1, 2), and in particular **in that** the connection is released during the movement of the two housing parts (10, 12) for moving the insertion device (5) and the puncture device (15) from the protected position to the insertion position, or during a subsequent movement of the two housing parts (10, 12) relative to each other.

17. Insertion head according to claim 16, **characterized in that** a first connection element (49) is formed on the base (1, 2) and a second connection element (16b) is formed on the housing (10, 12), **in that** the connection elements (49, 16b) engage with each other to create the connection, and **in that** at least one of the connection elements (16b) is movable out of the engagement counter to an elasticity force.

18. Insertion head according to one of claims 16 and 17, **characterized in that** a first connection element (49) and a second connection element (16b) engage with each other to create the connection, and one of the two housing parts (12), during its movement, contacts one of the connection elements (16b) and moves it out of said engagement counter to an elasticity force.

19. Insertion head according to claim 18, **characterized in that** one of the two housing parts (12) can be pushed so far into the other of the two housing parts (10) that, upon completion of the process of transferring the insertion device (5) and of the puncture device (15) from the protected position to the insertion position, a control ramp (52) on the first housing part (12) abuts against a deflector ramp (54) at a connection element (16) which connects the base (1, 2) reversibly to the housing (10, 12), and deflects it from an engagement position, such that the housing (10, 12) and the base (1, 2) are separable.

20. Insertion head according to claim 15 and according to one of claims 16 to 19, wherein the insertion head is formed such that a movement of the two housing parts (10, 12) relative to each other along a first distance (a) of a first portion (42a) of the guide slot (42) transfers the sliding block (44), guided by the guide slot (42), from a first position, which corresponds to the protected position of the insertion device (5) and of the puncture device (15), to a second position, which corresponds to the insertion position of the insertion device (5) and of the puncture device (15), wherein a connection device (16b) is provided that connects the base (1, 2) reversibly to the housing (10, 12), and wherein in order to release the housing (10, 12) from the base (1, 2), particularly precisely one of the two housing parts (12) is movable relative to the base (1, 2) along a second distance (b) perpendicular to the direction of extent of the insertion device (5) located in the insertion position, wherein the guide slot (42) has a second portion (42b) corresponding to the second distance (b).

21. Insertion head according to one of the preceding claims, **characterized in that**, during the movement of the insertion device (5) and of the puncture device (15) into the insertion position, the puncture device (15) connects to the housing (10, 12).

22. Insertion head according to one of the preceding claims, **characterized in that** the base (1, 2) forms a seat that receives the insertion device (5) and the puncture device (15) in their protected position.

23. Insertion head according to one of the preceding claims, **characterized in that** the housing (10, 12) forms a seat (14) that receives the insertion device (5) and the puncture device (15) in their protected position.

24. Insertion head according to one of the preceding claims, **characterized in that** the insertion device (5) is assigned a securing structure, which reversibly secures the insertion device (5) or puncture device (15) respectively in the protected position.

25. Insertion head according to claim 24, **characterized in that** the insertion device (5) is assigned a cannula housing (17b) which is moved along with the insertion device (5), which the cannula housing (17b) comprises a securing structure (46) which lies opposite a wall of one of the housing parts (12) extending parallel to the direction of movement of the two housing parts (10, 12) relative to each other, wherein an engagement element (48) on the inside face of this wall is in reversible engagement with the securing structure (46) in the protected position of the insertion device (5).

26. Insertion head according to one of the preceding claims, **characterized in that**, in one of the two housing parts (12) and/or in the other of the two housing parts (10) that receives the insertion device (5) and puncture device (15) in the protected position, a securing slot (50) is provided into which the insertion device (5) and puncture device (15) can be locked in the protected position.

27. Insertion head according to one of the preceding claims, **characterized in that** the puncture device (15) is assigned a retainer element (17a) which, during the automatic reverse movement of the puncture device (15) to a protected position, is moved along with the puncture device (15), wherein the retainer element (17a) has a locking shoulder (60) which, upon the automatic reverse movement in the housing (10, 12), comes into locking engagement with an engagement element (48), in order to prevent the puncture device (15) from moving out of the housing (10, 12) again.

## Revendications

1. Tête d'insertion pour applications médicales ou pharmaceutiques, comportant :
a) un boîtier (10, 12) avec une première partie (10) et une deuxième partie (12), la première et la deuxième partie étant mobiles l'une par rapport à l'autre ;
b) une base (1, 2) ;
c) un dispositif d'introduction (5) flexible, qui est stabilisé par un dispositif de perforation (15), le dispositif d'introduction (5) et le dispositif de perforation (15) étant montés mobiles conjointement ;
d) le dispositif d'introduction (5) et le dispositif de perforation (15) étant aptes à être déplacés par rapport au boîtier (10, 12) hors d'une position de protection, dans laquelle les extrémités libres du dispositif d'introduction (5) et du dispositif de perforation (15) sont disposées à l'intérieur des limites du boîtier (10, 12) et/ou de la base (1, 2), vers une position d'introduction, dans laquelle les extrémités libres s'avancent hors des limites du boîtier (10, 12) et/ou de la base (1, 2), de telle sorte qu'elles peuvent être introduites dans un tissu organique, et
e) un couplage (17a, 40, 42, 44), qui transforme le mouvement relatif des deux parties de boîtier (10, 12) de l'une par rapport à l'autre en un mouvement du dispositif d'introduction (5) et du dispositif de perforation (15), de telle sorte que ceux-ci peuvent être déplacés, sous l'effet du mouvement des deux parties de boîtier (10, 12) de l'une par rapport à l'autre, depuis la position de protection dans la position d'introduction,
la base (1, 2) comportant une face inférieure (U) qui est destinée à être positionnée sur le tissu organique dans la position d'introduction et sur laquelle s'avancent en saillie le dispositif d'introduction (5) et le dispositif de perforation (15), pour permettre une introduction de ceux-ci dans le tissu moyennant le positionnement de la base (1, 2) avec la face inférieure (U) sur le tissu, dans la position d'introduction, le dispositif de perforation (15) étant relié au boîtier (10, 12) et pouvant être désolidarisé du dispositif d'introduction (5) de telle sorte que, après l'introduction du dispositif d'introduction (5) et du dispositif de perforation (15) dans le tissu, le dispositif de perforation (15) peut être éloigné de la base (1, 2) et du dispositif d'introduction (5) en saisissant d'une main le boîtier (10, 12) et en déplaçant celui-ci à la main dans une direction opposée à la direction d'introduction conformément à sa destination, des moyens de rappel (65, 66) étant présent, par lesquels le dispositif de perforation (15), directement après son éloignement de la base (1, 2) et du dispositif d'introduction (5), peut être ramené automatiquement dans une position de protection, dans laquelle l'extrémité libre du dispositif de perforation (15) est disposée à l'intérieur des limites du boîtier (10, 12), le cas échéant, après qu'un organe de déclenchement ait été déclenché avec la main tenant le boîtier (10, 12).

2. Tête d'insertion selon la revendication 1, **caractérisée en ce que** les deux parties de boîtier (10, 12) peuvent être saisies entre les deux doigts d'une main, sachant que sous l'effet d'une pression appliquée par l'un des doigts sur l'une des parties de boîtier (10, 12), la deuxième partie de boîtier (12) peut être déplacée en direction de la première partie de boîtier (10).

3. Tête d'insertion selon l'une quelconque des revendications précédentes, **caractérisée en ce que** la base (1, 2) est assemblée de manière immobile au dispositif d'introduction (5), de telle sorte que lorsque celui-ci est déplacé de la position de protection dans la position d'introduction, ladite base est déplacée par rapport au boîtier (10, 12).

4. Tête d'insertion selon l'une quelconque des revendications 1 à 2, **caractérisée en ce que** la face inférieure (U) de la base (1, 2) forme au moins une partie d'une face extérieure du boîtier (10, 12), lorsque le dispositif d'introduction (5) et le dispositif de perforation (15) sont situés dans la position de protection, de telle sorte que, dans la position de protection, les extrémités libres de ces derniers sont en retrait derrière la face inférieure (U) de la base (1, 2), le dispositif d'introduction (5) et le dispositif de perforation (15) pouvant être déplacés par rapport à la base (1, 2), sous l'effet d'un mouvement de ceux-ci, depuis la position de protection dans la position d'introduction, de telle sorte que leurs extrémités libres dans la position d'introduction s'avancent au-delà de la face inférieure (U) de la base (1, 2).

5. Tête d'insertion selon la revendication 4, **caractérisée en ce que** le dispositif d'introduction (5) est monté mobile dans la base (1, 2).

6. Tête d'insertion selon la revendication 5, **caractérisée en ce que** la base (1, 2) et un élément d'articulation (6) ou un élément de guidage d'une coulisse de guidage forment conjointement une articulation ou une coulisse de guidage, et le dispositif d'introduction (5) forme saillie sur l'élément d'articulation (6) ou l'élément de guidage.

7. Tête d'insertion selon l'une quelconque des revendications 4 à 6, **caractérisée en ce que** le boîtier (10, 12) s'élève sur une face supérieure de la base (1, 2).

8. Tête d'insertion selon l'une quelconque des revendications précédentes, **caractérisée en ce que** les deux parties de boîtier (10, 12) peuvent pivoter ou coulisser l'une par rapport à l'autre, en particulier peuvent coulisser l'une dans l'autre, pour induire le mouvement du dispositif d'introduction (5) et du dispositif de perforation (15) depuis la position de protection dans la position d'introduction.

9. Tête d'insertion selon la revendication 8, **caractérisée en ce que** les deux parties de boîtier (10, 12) peuvent coulisser linéairement l'une par rapport à l'autre pour induire le mouvement du dispositif d'introduction (5) et du dispositif de perforation (15) depuis la position de protection dans la position d'introduction.

10. Tête d'insertion selon la revendication 4 et selon la revendication 9, **caractérisée en ce que** les deux parties de boîtier (10, 12) peuvent coulisser sensiblement parallèlement par rapport à la face inférieure (U) de la base (1, 2).

11. Tête d'insertion selon l'une quelconque des revendications précédentes, **caractérisée en ce que** le dispositif d'introduction (5) peut pivoter dans la position d'introduction.

12. Tête d'insertion selon l'une quelconque des revendications précédentes, **caractérisée en ce que** par le couplage (17a, 40, 42, 44), les deux parties de boîtier (10, 12), en particulier l'une des deux parties de boîtier (12), sont couplées avec le dispositif d'introduction (5), au moyen d'un engrènement, de préférence au moyen d'une roue dentée et d'une crémaillère.

13. Tête d'insertion selon la revendication 12, **caractérisée en ce que** l'une des deux parties de boîtier (12) est reliée à une crémaillère, qui est formée de préférence sur ladite partie de boîtier (12), et le dispositif d'introduction (5) est relié à une roue dentée située en engrènement avec la crémaillère.

14. Tête d'insertion selon la revendication 11 et selon l'une des revendications 12 à 13, **caractérisée en ce que** le dispositif d'introduction (5) est apte à pivoter autour d'un axe de rotation et est relié immobile en rotation à une roue dentée apte à tourner autour de l'axe de rotation et située dans l'engrènement.

15. Tête d'insertion selon l'une quelconque des revendications 1 à 11, **caractérisée en ce que** le dispositif d'introduction (5) et/ou le dispositif de perforation (15) sont maintenus par l'intermédiaire d'un axe de rotation (6, 40) sur la base (1) ou sur une première des deux parties de boîtier (10), un coulisseau (44) est présent, lequel est agencé de manière excentrée par rapport à l'axe de rotation (6, 40), une glissière (42) est présente, laquelle coopère de manière active avec le coulisseau (44), de telle sorte qu'un mouvement des deux parties de boîtier (10, 12) de l'une par rapport à l'autre, amène le coulisseau (44), guidé par la glissière (42), hors d'une première position, qui correspond à la position de protection du dispositif d'introduction (5) et du dispositif de perforation (15), dans une deuxième position qui correspond à la position d'introduction du dispositif d'introduction (5) et du dispositif de perforation (15).

16. Tête d'insertion selon l'une quelconque des revendications précédentes, **caractérisée en ce que** le boîtier (10, 12) est assemblé de manière amovible à la base (1, 2) et, en particulier, **en ce que** cet assemblage se désolidarise pendant le mouvement des deux parties de boîtier (10, 12) pour amener le dispositif d'introduction (5) et le dispositif de perforation (15) depuis la position de protection dans la position d'introduction ou pendant un autre mouvement des deux parties de boîtier (10, 12), l'une par rapport à l'autre, faisant suite à ce déplacement.

17. Tête d'insertion selon la revendication 16, **caractérisée en ce qu'**un premier élément d'assemblage (49) est formé sur la base (1, 2) et un deuxième élément d'assemblage (16b) est formé sur le boîtier (10, 12), **en ce que** les éléments d'assemblage (49, 16b) sont en prise l'un dans l'autre pour former l'assemblage, et **en ce qu'**au moins l'un des éléments d'assemblage (16b) peut être amené hors de prise à l'encontre d'une force élastique.

18. Tête d'insertion selon l'une des revendications 16 à 17, **caractérisée en ce qu'**un premier élément d'assemblage (49) et un deuxième élément d'assemblage (16b) sont conjointement en prise l'un dans l'autre pour former l'assemblage et l'une des deux parties de boîtier (12) pendant son mouvement entre en contact avec l'un des éléments d'assemblage (16b) et l'amène hors de prise à l'encontre d'une force élastique.

19. Tête d'insertion selon la revendication 18, **caractérisée en ce que** l'une des deux parties de boîtier (12) peut coulisser dans l'autre des deux parties de boîtier (10) sur une distance telle qu'une rampe de commande (52) sur la première partie de boîtier (12), à la fin du processus de déplacement du dispositif d'introduction (5) et du dispositif de perforation (15) hors de la position de protection dans la position d'introduction, vient buter contre une rampe de déviation (54) sur un élément d'assemblage (16), par lequel la base (1, 2) est assemblée de manière réversible au boîtier (10, 12), et dévie celui-ci hors d'une position de prise, de telle sorte que le boîtier (10, 12) et la base (1, 2) peuvent être séparés.

20. Tête d'insertion selon la revendication 15 et selon l'une quelconque des revendications 16 à 19, ladite tête d'insertion étant réalisée de telle sorte qu'un mouvement des deux parties de boîtier (10, 12) de l'une par rapport à l'autre sur une première distance (a) d'un premier tronçon (42a) de la glissière (42) amène le coulisseau (44), guidé par la glissière (42), hors d'une première position, qui correspond à la position de protection du dispositif d'introduction (5) et du dispositif de perforation (15), dans une deuxième position, qui correspond à la position d'introduction du dispositif d'introduction (5) et du dispositif de perforation (15), un dispositif d'assemblage (16b) étant prévu, par lequel la base (1, 2) est assemblée de manière réversible au boîtier (10, 12) et pour désolidariser le boîtier (10, 12) de la base (1, 2), en particulier exactement l'une des deux parties de boîtier (12) pouvant être déplacée par rapport à la base (1, 2), sur une deuxième distance (b) perpendiculairement à la direction d'extension du dispositif d'introduction (5) situé dans la position d'introduction, la glissière (42) comportant un deuxième tronçon (42b) correspondant à la deuxième distance (b).

21. Tête d'insertion selon l'une quelconque des revendications précédentes, **caractérisée en ce que** le dispositif de perforation (15) s'assemble au boîtier (10, 12) lorsque le dispositif d'introduction (5) et le dispositif de perforation (15) se déplacent dans la position d'introduction.

22. Tête d'insertion selon l'une quelconque des revendications précédentes, **caractérisée en ce que** la base (1, 2) forme un logement qui reçoit le dispositif d'introduction (5) et le dispositif de perforation (15) dans leur position de protection.

23. Tête d'insertion selon l'une quelconque des revendications précédentes, **caractérisée en ce que** le boîtier (10, 12) forme un logement (14) qui reçoit le dispositif d'introduction (5) et le dispositif de perforation (15) dans leur position de protection.

24. Tête d'insertion selon l'une quelconque des revendications précédentes, **caractérisée en ce qu'**une structure de sécurité est associée au dispositif d'introduction (5), par laquelle le dispositif d'introduction (5) ou le dispositif de perforation (15) sont bloqués de manière réversible dans la position de protection.

25. Tête d'insertion selon la revendication 24, **caractérisée en ce qu'**un boîtier de canule (17b) est associé au dispositif d'introduction (5), lequel est déplacé conjointement avec le dispositif d'introduction (5), ledit boîtier de canule (17b) comportant une structure de sécurité (46), qui est située en face d'une paroi de l'une des deux parties de boîtier (12), laquelle est parallèle à la direction du mouvement des deux parties de boîtier (10, 12) l'une par rapport à l'autre, un élément de prise (48), sur la face intérieure de ladite paroi, étant en prise réversible avec la structure de sécurité (46) dans la position de protection du dispositif d'introduction (5).

26. Tête d'insertion selon l'une quelconque des revendications précédentes, **caractérisée en ce que** dans l'une des deux parties de boîtier (12) et/ou dans l'autre des deux parties de boîtier (10), laquelle reçoit le dispositif d'introduction (5) et le dispositif de perforation (15) dans la position de protection, il est prévu une coulisse de sécurité (50), dans laquelle le dispositif d'introduction (5) et le dispositif de perforation (15) peuvent être arrêtés dans la position de protection.

27. Tête d'insertion selon l'une quelconque des revendications précédentes, **caractérisée en ce qu'**un élément de retenue (17a) est associé au dispositif de perforation (15), qui est entraîné en mouvement avec le dispositif de perforation (15) lorsque ledit dispositif de perforation (15) se déplace automatiquement en retour dans une position de protection, ledit élément de retenue (17a) comportant un épaulement d'arrêt (60) qui, au moment du retour automatique dans le boîtier (10, 12), entre en prise avec effet de blocage avec un élément de prise (48) pour empêcher que le dispositif de perforation (15) se déplace à nouveau hors du boîtier (10, 12).
